# EUROPEAN PATENT APPLICATION

(11) **EP 4 019 009 A1**
(43) Date of publication of application: **29.06.2022**
(21) Application number: 22157319.9
(22) Date of filing: 07.03.2018
(51) Int. Cl.: A61K 9/20, A61K 31/4152, A61P 7/00

(54) **PHARMACEUTICAL TABLET COMPOSITION COMPRISING ELTROMBOPAG OLAMINE**

(30) Priority: 26.04.2017 IN 201711014792; 14.07.2017 EP 17181421
(62) Divisional of application: 18711260.2
(71) Applicant: Alfred E. Tiefenbacher (GmbH & Co. KG), 22767 Hamburg (DE)
(72) Inventor: STAVER, Ruslan, 22767 Hamburg (DE); PRATHAP, Vamshi Ramana, 502 319 Telangana (IN); VADLA, Hari Kiran Chary, 502 319 Telangana (IN); RALLABANDI, Bala Ramesha Chary, 502 319 Telangana (IN); SCHLEHAHN, Hendrik, 22767 Hamburg (DE)
(74) Representative: Prinz & Partner mbB

(57) **Abstract**

The present invention relates to a pharmaceutical tablet composition comprising eltrombopag olamine and one or more reducing sugars, a production process therefore, a pharmaceutical tablet composition comprising eltrombopag olamine and one or more reducing sugars obtainable by the production process, a use / method of use of the pharmaceutical tablet compositions in the treatment or prophylaxis of immune (idiopathic) thrombocytopenic purpura (ITP), thrombocytopenia and/or acquired severe aplastic anaemia (SAA).

## Description

### Technical Field:

The present invention relates to a pharmaceutical tablet composition comprising eltrombopag olamine and one or more reducing sugars, a production process therefore, a pharmaceutical tablet composition comprising eltrombopag olamine and one or more reducing sugars obtainable by the production process, a use / method of use of the pharmaceutical tablet compositions in the treatment or prophylaxis of immune (idiopathic) thrombocytopenic purpura (ITP), thrombocytopenia and/or acquired severe aplastic anaemia (SAA).

### Background of the invention:

3'-(N'-[1-(3,4-dimethylphenyl)-3-methyl-5-oxo-1,5-dihydropyrazol-4-ylidene]hydrazino}-2'-hydroxy-biphenyl-3-carboxylic acid - 2-aminoethanol (1:2) (hereinafter and in the context of the present invention called eltrombopag olamine, CAS 496775-62-3) is a non-peptide haematopoietic receptor agonist, which mimics haematopoietic growth factors, including thrombopoietin (TPO), particularly enhancing platelet production and, thus, is particularly useful in the treatment of thrombocytopenia (see e.g. WO 03/098992 A2 and WO 2008/136843A1).

In Europe eltrombopag olamine is approved under the tradename Revolade^{®} comprising eltrombopag olamine in an amount corresponding to 12.5 mg, 25 mg, 50 mg, and 75 mg eltrombopag free acid in tablet form. In USA eltrombopag olamine is approved under the tradename Promacta^{®} comprising eltrombopag olamine in an amount corresponding to 12.5 mg, 25 mg, 50 mg, 75 mg and 100 mg eltrombopag free acid in tablet form. Revolade^{®} tablets are packed in Alu-Alu blisters, whereas Promacta^{®} tablets are packed in HDPE bottles.

In Europe, Revolade^{®} is indicated for adult chronic immune (idiopathic) thrombocytopenic purpura (ITP) splenectomised patients who are refractory to other treatments (e.g. corticosteroids, immunoglobulins). Revolade^{®} may be considered as second line treatment for adult non-splenectomised patients where surgery is contraindicated.

Revolade^{®} is also indicated in adult patients with chronic hepatitis C virus (HCV) infection for the treatment of thrombocytopenia, where the degree of thrombocytopenia is the main factor preventing the initiation or limiting the ability to maintain optimal interferon-based therapy.

Revolade^{®} is furthermore indicated in adult patients with acquired severe aplastic anaemia (SAA) who were either refractory to prior immunosuppressive therapy or heavily pretreated and are unsuitable for haematopoietic stem cell transplantation.

In WO 03/098992 A2 an eltrombopag tablet composition is disclosed in example 6 which comprises in addition to 8.45 mg eltrombopag olamine, furthermore 112 mg microcrystalline cellulose, 70 mg lactose, 8 mg sodium starch glycolate and 2 mg magnesium stearate.

It is, however, known, that eltrombopag olamine undergoes a Maillard reaction with respective pharmaceutically acceptable excipients, such as reducing sugars, e.g. lactose. It is known that eltrombopag olamine is degraded in presence of lactose and forms impurities, which can be measured in the pharmaceutical tablet composition. Mr. Kapsi, one of the inventors of WO 2008/136843 A1 provided in the corresponding US examination of US 2010/0040684 A1 a declaration (in the context of the present application called "Kapsi declaration") disclosing experimental stability data for the use of the lactose containing eltrombopag tablet composition of example 6 in WO 03/098992 A2 in comparison to a tablet formulation, which is free of reducing sugars using mannitol instead.

According to the Kapsi declaration, the eltrombopag olamine tablet formulation comprising lactose shows an "*increase in degradation products when compared to the mannitol based formulation (4 fold difference in degradation products at 3 months*" (see section Results on page 1). In section 5 on page 5 of the Kapsi declaration Mr. Kapsi followed that the "*experimental data described herein demonstrates that the formulation described* as *Example 6 in publication WO 03*/*098992 A2 leads to a tablet formulation with a four-fold higher level of degradation products at 3 months and in my experience leads to an unacceptable tablet formulation".*

In order to avoid unacceptable degradation of eltrombopag olamine, WO 2008/136843 A1 teaches to use diluents substantially free of reducing sugars, in particular using mannitol. The avoidance of reducing sugars as pharmaceutical excipient in an eltrombopag olamine pharmaceutical tablet is continued in WO 2012/121957 A1 and WO 2015/0121957 A2.

It would, however, be desirable to provide an alternative solution for a stable eltrombopag olamine pharmaceutical tablet composition, wherein the degradation level of eltrombopag olamine is negligible and the pharmaceutical tablet composition fulfills the regulatory stability criteria for pharmaceutical tablets. Furthermore, it would be desirable that the pharmaceutical tablet composition is more economic in costs per unit and/or allows tuning one or more pharmaceutical technological aspects during the production process.

### Brief description of the invention:

One or more problems of the present invention is/are solved by the subjects of the independent claims. Advantages (preferred embodiments) are set out in the detailed description hereinafter as well as in the dependent claims.

Accordingly, a first aspect of the present invention relates to a pharmaceutical tablet composition comprising eltrombopag olamine as active ingredient and one or more pharmaceutically acceptable excipients including one or more reducing sugars, characterized in that the composition comprises or consists of
either
a) eltrombopag olamine in a therapeutically effective amount and one or more pharmaceutically acceptable excipients in an intragranular composition, and
b) one or more reducing sugars, preferably selected from the group consisting of lactose, maltose, glucose, arabinose, and fructose and optionally further one or more pharmaceutically acceptable excipients in an extragranular composition
   or
c) one or more reducing sugars, preferably selected from the group consisting of lactose, maltose, glucose, arabinose, and fructose and optionally further one or more pharmaceutically acceptable excipients in an intragranular composition and
d) eltrombopag olamine in a therapeutically effective amount and one or more pharmaceutically acceptable excipients in an extragranular composition.

A second aspect of the present invention relates to an inventive pharmaceutical tablet composition for use in the treatment or prophylaxis of immune (idiopathic) thrombocytopenic purpura (ITP), thrombocytopenia and/or acquired severe aplastic anaemia (SAA).

A third aspect of the present invention relates to a process of production of an inventive pharmaceutical tablet comprising eltrombopag olamine as active ingredient and one or more pharmaceutically acceptable excipients including one or more reducing sugars, characterized in that the process comprises the following steps:
either
a. mixing a therapeutically effective amount of eltrombopag olamine and one or more pharmaceutically acceptable excipients,
b. wet granulation of components mixed in step a) with a suitable granulation fluid, preferably water or an aqueous solution of a binder, in a suitable amount to form an intragranular composition,
c. mixing one or more reducing sugars, preferably selected from the group consisting of lactose, maltose, glucose, arabinose, and fructose and optionally further one or more pharmaceutically acceptable excipients to form an extragranular composition,
d. mixing the extragranular composition of step c) with the intragranular composition of step b) to form a tablet composition, and
e. compressing the tablet composition of step d) to form the pharmaceutical tablet composition comprising eltrombopag olamine as active ingredient in the intragranular phase and one or more reducing sugars in the extragranular phase,
   or
f. mixing one or more reducing sugars, preferably selected from the group consisting of lactose, maltose, glucose, arabinose, and fructose, preferably lactose and optionally further one or more pharmaceutically acceptable excipients,
g. wet granulation of components mixed in step f) with a suitable granulation fluid, preferably water or an aqueous solution of a binder, to form an intragranular composition,
h. mixing a therapeutically effective amount of eltrombopag olamine and one or more pharmaceutically acceptable excipients to form an extragranular composition,
i. mixing the extragranular composition of step h) with the intragranular composition of step g) to form a tablet composition, and
j. compressing the tablet composition of step i) to form the pharmaceutical tablet composition comprising eltrombopag olamine as active ingredient in the extragranular phase and one or more reducing sugars in the intragranular phase.

A fourth aspect of the present invention relates to a pharmaceutical tablet composition comprising eltrombopag olamine as active ingredient and one or more pharmaceutically acceptable excipients including one or more reducing sugars, characterized in that the composition which comprises or consists of either
a) eltrombopag olamine in a therapeutically effective amount and one or more pharmaceutically acceptable excipients in an intragranular composition, and
b) one or more reducing sugars, preferably selected from the group consisting of lactose, maltose, glucose, arabinose, and fructose and optionally further one or more pharmaceutically acceptable excipients in an extragranular composition,
   or
c) one or more reducing sugars, preferably selected from the group consisting of lactose, maltose, glucose, arabinose, and fructose and optionally further one or more pharmaceutically acceptable excipients in an intragranular composition and
d) eltrombopag olamine in a therapeutically effective amount and one or more pharmaceutically acceptable excipients in an extragranular composition
is obtainable by the inventive production process.

A fifth aspect of the present invention relates to a use of an inventive pharmaceutical tablet composition, wherein the pharmaceutical tablet composition is used in the manufacture of a medicament for the treatment or prophylaxis of immune (idiopathic) thrombocytopenic purpura (ITP), thrombocytopenia and/or acquired severe aplastic anaemia (SAA).

A sixth aspect of the present invention relates to a method of treatment or prophylaxis of immune (idiopathic) thrombocytopenic purpura (ITP), thrombocytopenia and/or acquired severe aplastic anaemia (SAA), comprising administering to a patient in need an inventive pharmaceutical tablet composition.

The inventive aspects of the present invention as disclosed hereinbefore can comprise - in case it is reasonable for a person skilled in the art - any possible combination of the preferred inventive embodiments as set out in the dependent claims or disclosed in the following detailed description.

### Detailed description of the invention:

It has surprisingly been found by the present inventors, that the inventive pharmaceutical tablet comprising eltrombopag olamine in one granular composition (either intra- or extragranular) and one or more reducing sugars in a different granular composition (either extra- or intragranular) is not subjected to a substantial degradation of the active ingredient eltrombopag olamine in view of Maillard reactions with the reducing sugar and, thus, fulfills the regulatory stability requirements for commercializing the pharmaceutical eltrombopag olamine tablet (see example section Part E, tables 1 and 2). According to a preferred embodiment of the present invention, the inventive pharmaceutical tablet is stable over at least 6 months when stored preferably in Alu-Alu blisters at intermediate or long-term storage conditions, preferably at 25°C / 60% relative humidity (RH) and/or 40°C / 75 % RH. The inventive pharmaceutical tablet composition according to all aspects of the present invention comprises reducing sugars only in that granular composition (phase), which does not comprise the eltrombopag olamine constituent. Thus, in case eltrombopag olamine is present in the intragranular composition, the reducing sugar is present in the extragranular composition and in case the eltrombopag olamine is present in the extragranular composition, the reducing sugar is present in the intragranular composition.

Furthermore, the experimental data provided in table 3 in Part E) of the example section shows that 75 wt.% or more, preferably more than 80 wt.%, more preferably more than 90 wt.% of eltrombopag olamine in the inventive pharmaceutical tablet composition is dissolved within 45 minutes in accordance with the regulatory standard test.

The finding of the present inventors is moreover surprising, as it has been disclosed in the so called Kapsi declaration that eltrombopag olamine compositions comprising lactose are subject to severe degradation and, thus, do not fulfill the stability requirements for commercializing the respective pharmaceutical eltrombopag olamine tablet. The originator accordingly exchanged the lactose excipient by a non-reducing sugar, namely mannitol to fulfill the stability requirements.

Thus, by use of the inventive teaching, namely by separating the eltrombopag olamine active ingredient and the reducing sugar in different granular compositions, it is possible to maintain the use of a reducing sugar as diluent constituent in the tablet formulation of eltrombopag olamine and at the same time achieve a suitable dissolution profile for the inventive pharmaceutical tablet composition.

In the context of the present invention the term "intragranular composition" refers to tablet constituents which are wet granulated and thus forming granules to be further processed as intragranular composition / phase of the inventive pharmaceutical tablet composition. In case the intragranular tablet constituents comprise a therapeutically effective amount of eltrombopag olamine and one or more pharmaceutically acceptable excipients, then the intragranular composition does not comprise a reducing sugar, e.g. lactose, as pharmaceutically acceptable excipient. Alternatively, in case the intragranular composition comprises a reducing sugar, e.g. lactose and optionally one or more pharmaceutically acceptable excipients, then the intragranular composition does not comprise eltrombopag olamine.

The "extragranular composition" is admixed with the granules of the intragranular composition and the mixture forms the basis for the tablet composition. This mixture is subsequently compressed to form the inventive pharmaceutical tablet composition. In case the extragranular composition comprises or consists of one or more reducing sugars and optionally one or more further pharmaceutical excipients then the extragranular composition does not comprise eltrombopag olamine. Alternatively, in case the extragranular composition comprises eltrombopag olamine and optionally one or more pharmaceutically acceptable excipients, then the extragranular composition does not comprise a reducing sugar, in particular lactose.

In the context with all inventive aspects of the present invention, the inventive pharmaceutical tablet composition comprises eltrombopag olamine as the active ingredient in a therapeutically effective amount and optionally one or more further active ingredients. In the context of all aspects and preferred embodiments of the present invention, the term "eltrombopag olamine" refers to an amorphous form or one or more polymorphic crystalline forms of eltrombopag olamine, wherein eltrombopag olamine has been defined hereinbefore as eltrombopag bisethanolamine. The amorphous as well as polymorphic forms I, II, and III of eltrombopag olamine have been disclosed, e.g., in WO 2010/114943 A1. More preferably, eltrombopag olamine in particular polymorphic form I of eltrombopag olamine (cf. figure 28 of WO 2010/114943 A1 shows polymorphic form I of eltrombopag bisethanolamine) is used in accordance with all aspects and preferred embodiments of the present invention. Furthermore, the eltrombopag olamine is generally used exhibiting suitable particle sizes for formulating the inventive pharmaceutical tablet composition, wherein preferably the particle size distribution by volume (PSD) is 30 µm or less for 90 Vol. % of the particles (D 0.9), 8 µm or less for 50 Vol. % of the particles (D 0.5), and/or 2 µm or less for 10 Vol. % of the particles (D 0.1). The particle size distribution by volume may be determined by use of the Malvern technique (by volume). In preferred embodiments of all aspects of the present invention, the active ingredient eltrombopag olamine is used per tablet unit in an amount of 16.05 mg, 32.1 mg, 64.2 mg, 96.3 mg or 128.4 mg eltrombopag olamine (respectively corresponding to 12.5 mg, 25 mg, 50 mg, 75 mg or 100 mg eltrombopag free acid).

In view of eltrombopag olamine as active ingredient, the inventive pharmaceutical tablet composition is effective for use in the treatment or prophylaxis of immune (idiopathic) thrombocytopenic purpura (ITP), thrombocytopenia and/or acquired severe aplastic anaemia (SAA).

Preferably the inventive pharmaceutical tablet composition is indicated for adult chronic immune (idiopathic) thrombocytopenic purpura (ITP) in splenectomised patients who are refractory to other treatments (e.g. corticosteroids, immunoglobulins). The inventive pharmaceutical tablet composition may also be considered as second line treatment for adult non-splenectomised patients where surgery is contraindicated.

The inventive pharmaceutical tablet composition is preferably furthermore indicated in adult patients with chronic hepatitis C virus (HCV) infection for the treatment of thrombocytopenia, where the degree of thrombocytopenia is the main factor preventing the initiation or limiting the ability to maintain optimal interferon-based therapy.

The inventive pharmaceutical tablet composition is preferably furthermore indicated in adult patients with acquired severe aplastic anaemia (SAA) who were either refractory to prior immunosuppressive therapy or heavily pretreated and are unsuitable for haematopoietic stem cell transplantation.

According to all aspects of the present invention suitable reducing sugars, i.e. sugar constituents which generally may form a Maillard reaction with the active ingredient eltrombopag olamine, are used as the one or more reducing sugars acting as diluents in the extragranular composition of the inventive pharmaceutical tablet composition. In an alternative or cumulative preferred embodiment of the present invention the one or more reducing sugars are selected from the group consisting of lactose, maltose, glucose, arabinose and fructose, more preferably the one or more reducing sugar comprises or consists of lactose selected from the group consisting of lactose monohydrate, anhydrous lactose, spray dried lactose and co-processed lactose. Examples of co-processed lactose are Ludipress^{®} (lactose monohydrate 93 wt.%, 3.5 wt.% povidone (Kollidon^{®}30) and 3.5 wt.% crospovidone (Kollidon^{®} CL); Cellactose^{®} (75% lactose and 25% microcrystalline cellulose MCC); Starlac^{®} (85% lactose and 15% starch) and Combilac^{®} (70% lactose, 20% MCC and 10% corn starch).

The use of lactose as diluent in the tablet composition is advantageous, as lactose is in particular cheaper than other excipients and thus allows a more economic production of pharmaceutical eltrombopag olamine tablet compositions. Furthermore the use of lactose is advantageous, as a variety of commercially available grades (lactose monohydrate, anhydrous lactose and spray-dried lactose) exists, which enables the skilled person to choose the best fitting grade for the present situation. Accordingly, the skilled person is enabled with respect to all aspects of the present invention to select the best fitting lactose excipient based on the required characteristics for tablet compression. For example, in case the lactose excipient shall also facilitate non-sticking at the compression facilities, lactose comprising a comparatively large particle size will be selected. In case lactose monohydrate is used, fine grades are usually used for the wet granulation process, as they permit better mixing with other excipients.

In accordance with the present invention, the reducing sugar content in the granular composition not comprising the eltrombopag olamine, thus, either the extra- or intragranular composition of the inventive pharmaceutical tablet composition is preferably greater than 5 wt.%, more preferably at least 9 wt.%, even more preferably in the range of 15 to 75 wt.%, e.g., 20 to 25 wt.%, 30 to 35 wt.%, 36 to 39 wt.%, 40 to 44 wt.%, 45 to 51 wt.%, 52 to 57 wt.%, 58 to 65 wt.% or alternatively 70 to 75 wt.%, respectively based on the total weight of the pharmaceutical tablet composition. According to an alternative embodiment of the present invention, the inventive pharmaceutical eltrombopag olamine tablets is made using at least 33 to 38 wt.% lactose based on the total weight of the tablet. According to a preferred embodiment the inventive pharmaceutical eltrombopag tablet, the relative content of lactose in comparison to the total weight of the tablet increases with reducing the relative content of the active ingredient eltrombopag olamine. Accordingly, an inventive tablet composition comprising 95.7 mg eltrombopag olamine (equivalent to 75 mg eltrombopag free acid) per tablet unit comprises, e.g. 33 to 38 wt.%, preferably 35 wt.% lactose based on the total weight of the tablet. An inventive tablet composition comprising 63.8 mg eltrombopag olamine (equivalent to 50 mg eltrombopag free acid) per tablet unit comprises, e.g. 45 to 51 wt.%, preferably 47 wt.% lactose based on the total weight of the tablet. An inventive tablet composition comprising 31.9 mg eltrombopag olamine (equivalent to 25 mg eltrombopag free acid) per tablet unit comprises, e.g. 55 to 62 wt.%, preferably 59 wt.% lactose based on the total weight of the tablet. An inventive tablet composition comprising 16 mg eltrombopag olamine (equivalent to 12.5 mg eltrombopag free acid) per tablet unit comprises, e.g. 58 to 65 wt.%, preferably 61 wt.% lactose based on the total weight of the tablet.

According to an alternative or cumulative preferred embodiment of all aspects of the present invention, the intragranular optional extragranular composition of the inventive pharmaceutical tablet composition further comprises or consists of one or more suitable binder agents, preferably one or more binder agents selected from the group consisting of povidone (non-crosslinked polyvinyl pyrrolidone, also called PVP, e.g. commercially available as Plasdone K29/32 or Kollidon^{®} 30), copovidone (vinylpyrrolidone-vinyl acetate copolymer in a ratio of 3:2 by mass, also called PVP/VA, e.g. commercially available as Kollidon^{®} VA64), hydroxypropyl cellulose (also called HPC or hyprollose, e.g. commercially available as Klucel^{™} ELF), low-substituted hydroxypropyl cellulose, hydroxypropyl methylcellulose (also called HPMC or hypromellose, e.g. commercially available as Methocel^{™} K3 Premium LV), hydroxypropyl starch (also called HPS), polyethylene oxide, more preferably one or two binder agents are selected from povidone and copovidone. According to a preferred embodiment, the low-substituted hydroxypropyl cellulose is preferably used based on its disintegrant properties. Furthermore it is preferred to use polyethylene oxide with molecular weight below 9000 g/mol, which is usually named polyethylene glycol (also called PEG or Macrogol, e.g. commercially available as Carbowax^{™} PEG). Alternatively or cumulatively preferred are one or more constituents of the group consisting of polyvinyl alcohol (e.g. commercially available as PVA Emprove^{®} 4-88), , polyvinyl caprolactam-polyvinyl acetate-polyethylene glycol graft copolymer (e.g. commercially available as Soluplus^{®}), polyethylene glycol-polyvinyl alcohol graft copolymer (also called PEG-PVA, e.g. commercially available as Kollicoat^{®} IR), and sodium carboxymethyl cellulose (also called Na-CMC, e.g. commercially available as Walocel^{™} CRT). The use of one or more binder agents is in particular preferred in the intragranular composition. In case co-processed lactose comprising binder agents are used in the extragranular phase, then the amount of binder agent in the intragranular composition may be reduced (see also below paragraph). The additional use of one or more binder agents in the intragranular composition and optionally also in the extragranular composition is preferred, as the respective inventive tablet compositions show a reduced degradation of eltrombopag olamine at higher amounts of 9 wt.% or more, preferably 20 wt.% or more of reducing sugars, in particular lactose as set out in the example section.

The above mentioned suitable binder agents are generally classified as "semisynthetic polymeric binder agents" or "synthetic polymeric binder agents".

In the context of the present invention the term "binder" is defined as an agent used to increase the cohesion of the powdery particles or granules during the compression, in order to obtain pharmaceutical forms with a defined hardness, or to act as processing aid during the granulation process. The binder may be present in the pharmaceutical composition in the form of a single constituent / ingredient or in the form of a mixture of constituents / ingredients. In the context of the present invention the binder agents are classified into 3 classes: 1) "natural binder", 2) "semisynthetic polymeric binder", and 3) "synthetic polymeric binder".

In the context of the present invention the expression "semisynthetic polymeric binder" refers to a chemically modified natural polymer binder, usually a derivative of cellulose or starch, preferably selected from the group consisting of hydroxypropyl cellulose (HPC, hyprollose), hydroxypropyl methyl cellulose (HPMC, hypromellose), sodium carboxymethyl cellulose, and hydroxypropyl starch.

In the context of the present invention the expression "synthetic polymeric binder" refers to a fully chemically synthesized, human or machine made, non-natural polymer or co-polymer binder agent, preferably selected from the group consisting of povidone, copovidone, polyvinyl alcohol, polyethylene glycol (PEG), polyvinyl caprolactam-polyvinyl acetate-polyethylene glycol graft copolymer, and polyethylene glycol-polyvinyl alcohol graft copolymer (PEG-PVA).

In the context of the present invention the expression "polymeric binder" / "polymer binder" refers to a group of polymeric binder agents consisting of "semisynthetic polymeric binders" and "synthetic polymeric binders", as defined above.

According to an alternative or cumulative preferred embodiment of all aspects of the present invention, the inventive pharmaceutical tablet composition may further comprise suitable natural binder agents, preferably selected from the group consisting of starch or starch derivatives, e.g., corn starch, potato starch, pregelatinized starch, sodium starch; alginic acid or salts thereof, e.g. sodium alginate; gelatin, Guar gum; gum Arabic; Candelilla wax; and Carnauba wax.

Starch and pregelatinized starch (PGS) are multifunctional excipients. They can be considered as natural binders, but they can be used also as diluents, and furthermore also have disintegrant-like properties. A disintegrant is typically an agent used in the preparation of solid pharmaceutical formulations which causes them to disintegrate and release their medicinal substances on contact with moisture. However, the disintegrant may also be considered as a diluent and/or binder, depending on the properties of the particular excipient employed as a disintegrant. A binder is typically a substance used to create a desired consistency in a formulation, whereby a diluent is typically considered as a bulking agent to increase the mass of the formulation. Accordingly, the disintegrant exhibits properties of disintegration upon contact with aqueous environments, and is preferably a non-soluble disintegrant, but may also show properties associated with a diluent or binder. PGS is a starch that has been chemically and/or mechanically processed to rupture all or part of the starch granules. This typically renders the starch flowable and directly compressible (Handbook of Pharmaceutical Excipients (Ed: Rowe)). Partially pregelatinized starch is commercially available. In some embodiments, pregelatinized starch contains 5% of free amylose, 15% of free amylopectin, and 80% unmodified starch. Pregelatinized starch is typically obtained from maize (corn), potato, or rice starch.

According to an alternative or cumulative preferred embodiment of the present invention, the use of co-processed lactose is preferred, as it already comprises a mixture of lactose and one or more further excipients. The one or more further excipients in the co-processed lactose are preferable selected from microcrystalline cellulose (MCC), starch, povidone, and crospovidone (crosslinked polyvinyl pyrrolidone, so called PVPP or polyvinyl polypyrrolidone, e.g. commercially available as Polyplasdone XL or Kollidon^{®} CL). Commercially available co-processed lactose is available as, e.g., Ludipress^{®} which comprises of lactose, povidone (Kollidon^{®} 30) and crospovidone (Kollidon^{®} CL), Cellactose^{®} (lactose with MCC); Starlac^{®} (lactose with starch) and Combilac^{®} (lactose with MCC and starch). In case the further excipients of the co-processed lactose is also present as separate excipient in a different granular phase, then the amount of the separate excipient of the other granular phase may be reduced.

According to an alternative or cumulative preferred embodiment of all aspects of the present invention, the intragranular and/or extragranular composition, preferably the intragranular and extragranular composition further comprises suitable further diluent agents (in addition to the one or more reducing sugars), preferably one or more further diluent agents are selected from the group consisting of erythritol, isomalt, maltitol, xylitol, microcrystalline cellulose, powdered cellulose, pregelatinized starch, starch, lactitol, mannitol, sorbitol, maltodextrin more preferably one, two or more further diluent agents are selected from erythritol, isomalt, maltitol, xylitol, and microcrystalline cellulose, even more preferably the further diluent agents are selected from microcrystalline cellulose and one, two or more selected from erythritol, isomalt, maltitol and xylitol, preferably xylitol.

According to an alternative or cumulative preferred embodiment of all aspects of the present invention, the inventive tablet composition comprises in addition to a reducing sugar in the respective granular composition, i.e. either the extra- or the intragranular composition one or more, preferably one additional diluent agent as set out above. Such a combination is in particular preferred for inventive tablet compositions with a low content of eltrombopag olamine, e.g. 16.05 mg, 32.1 mg, 64.2 mg.

According to an alternative or cumulative preferred embodiment of all aspects of the present invention it may be preferred that the composition comprising the eltrombopag olamine, i.e. either the intra- or extragranular composition comprises one or more, preferably two further diluent agents as set out above, preferably comprising microcrystalline cellulose and one of the further diluent agents as set out above. Such a combination is preferred as the use of microcrystalline cellulose alone as diluent agent in the respective phase, preferably the intragranular phase may reduce the disintegration time of the inventive tablet composition over storing time.

According to an alternative or cumulative preferred embodiment of all aspects of the present invention it may be preferred that a combination of diluent agents comprises one or more, preferably one soluble and one or more, preferably one insoluble diluent agent in the respective granular phases/compositions. Such a combination further facilitates the disintegration of the inventive tablet, in particular in case no further in particular intragranular disintegrant agent is used in the inventive tablet. Preferred examples of such an inventive combination comprise microcrystalline cellulose as insoluble diluent and lactose or xylitol as soluble diluents.

According to an alternative or cumulative preferred embodiment of all aspects of the present invention, the intragranular and/or extragranular composition, preferably the extragranular composition further comprises suitable one or more disintegrant agents, preferably one or more disintegrant agents selected from the group consisting of sodium starch glycolate, crospovidone, and croscarmellose sodium, more preferably one or two selected from sodium starch glycolate, crospovidone and croscarmellose sodium. Alternatively, low-substituted hydroxypropyl cellulose (L-HPC) is used as disintegrant agent. Generally the disintegrant is applied in the extragranular composition. In case, however, the inventive tablet composition comprises a higher amount of eltrombopag olamine in the intragranular phase and/or an insoluble diluent can be used in the intragranular phase in addition to eltrombopag olamine, then the same or different disintegrant agent may be used also in the intragranular composition, wherein the weight ratios of disintegrant in intra- and extragranular phase is preferably in the range of 1:1 to 1:5, in particular 1:3 or 1:4 or 1:4.6.

As an example, the intragranular phase may comprise no disintegrant agent and the extragranular phase may comprise sodium starch glycolate, crospovidone or croscarmellose sodium or a combination of sodium starch glycolate and crospovidone or sodium starch glycolate and croscarmellose sodium or crospovidone and croscarmellose sodium, more preferably sodium starch glycolate as disintegrant agent(s) or alternatively low-substituted hydroxypropyl cellulose as disintegrant, wherein the disintegrant agent in total in the extragranular phase is in particular present in a range of 5 to 10 wt.%, preferably 8 wt.% based on the total weight of the inventive tablet composition.

As an alternative example, the intragranular phase may comprise sodium starch glycolate, crospovidone or croscarmellose sodium or a combination of sodium starch glycolate and crospovidone or sodium starch glycolate and croscarmellose sodium or crospovidone and croscarmellose sodium, more preferably sodium starch glycolate as disintegrant agent(s) or alternatively low-substituted hydroxypropyl cellulose as disintegrant, wherein the disintegrant agent in total in the intragranular phase is in particular present in a weight range of 1 to 5 wt.%, preferably 2 wt.% based on the total weight of the inventive tablet composition, while the extragranular phase may comprise sodium starch glycolate, crospovidone or croscarmellose sodium, or a combination of sodium starch glycolate and crospovidone or sodium starch glycolate and croscarmellose sodium or crospovidone and croscarmellose sodium or alternatively low-substituted hydroxypropyl cellulose, wherein the disintegrant agent in total in the extragranular phase is in particular present in a range of 5 to 10 wt.%, preferably 8 wt.% based on the total weight of the inventive tablet composition.

According to an alternative or cumulative preferred embodiment of all aspects of the present invention, the extragranular composition of the inventive pharmaceutical tablet composition further comprises one or more suitable lubricant agents, preferably selected from the group consisting of magnesium stearate, stearic acid, and sodium stearyl fumarate, more preferably magnesium stearate.

According to a further alternative or cumulative preferred embodiment of all aspects of the present invention the pharmaceutical tablet composition is film coated with a coating agent, preferably comprising one or more pharmaceutically acceptable polymers, optionally one or more pharmaceutically acceptable plasticizers, and optionally one or more pharmaceutically acceptable pigments. The one or more polymer agents of the pharmaceutically acceptable coating agent are preferably selected from one or more of the group consisting of hydroxypropyl cellulose, hydroxypropylmethyl cellulose, ethyl cellulose, polyvinyl alcohol, polyethylene glycol, polyethylene glycol-polyvinyl alcohol graft copolymer, and polysorbate. The one or more pigments are preferably selected from the group of titanium dioxide, aluminium lakes, and iron oxides, preferably iron oxide yellow, iron oxide red, and iron oxide black.

According to an alternative or cumulative preferred embodiment of all aspects of the present invention the coating agent comprises or consists of
- hypromellose, macrogol 400, polysorbate 80 and titanium dioxide (commercially available as Opadry^{®} White), preferably for inventive pharmaceutical tablet compositions comprising 16.05 mg or 32.1 mg eltrombopag olamine, or
- hypromellose, macrogol 400, titanium dioxide, iron oxide yellow and iron oxide red (commercially available as Opadry^{®} Brown), preferably for inventive pharmaceutical tablet compositions comprising 64.2 mg eltrombopag olamine, or
- hypromellose, macrogol 400, titanium dioxide, iron oxide red and iron oxide black (commercially available as Opadry^{®} Brown), preferably for inventive pharmaceutical tablet compositions comprising 96.3 mg eltrombopag olamine.

According to alternative or cumulative preferred embodiments of all aspects of the present invention,
i. the total content of binder agent(s) is at least 0.5 wt.% or more, more preferably is in the range of 0.5 to 7 wt.%, more preferably 1 to 3 wt.% and/or
ii. the total content of diluent agent(s) including the reducing sugar amount is at least 5 wt.% or more, preferably is present in the range of 10 to 86 wt.%, alternatively in the range of 15 to 80 wt.%, alternatively in the range of 7 to 62 wt.%, alternatively in the range of 63 wt.% to 86 wt.% and/or
iii. the total content of the disintegrant agent(s) is at least 3 wt.% or more, preferably is in the range of 4 to 15 wt.%, more preferably 5 to 14 wt.%, even more preferably 6 to 12 wt.% or alternatively is in the range of 3 to 5 wt.% and/or
iv. the total content of the lubricant agent(s) is at least 0.5 wt.% or more, preferably is present in the range of 0.5 to 5 wt.%, preferably 0.8 to 3 wt.%, more preferably 0.9 to 1.5 wt.% and more preferably is 1 wt.% and/or
v. the total content of the coating agent(s) is at least 2 wt.%, preferably in the range of 3 to 7 wt.%, more preferably 4 to 5 wt.%,
respectively based on the total weight of the pharmaceutical tablet composition.

According to one embodiment of the present invention where the inventive tablet composition comprises 96.3 mg eltrombopag olamine (corresponding to 75 mg eltrombopag free acid) in the intragranular composition, the intragranular composition comprises in addition to eltrombopag olamine at least one binder agent as set out above in an amount of at least 1 wt.% or more, more preferably in the range of 1 wt.% to 5 wt.%, more preferably in the range of 2 wt.% to 4 wt.% based on the total weight of the produced pharmaceutical tablet composition, more preferably the binder agent is povidone. Furthermore, the intragranular composition comprises one, two or more further diluent agents as set out above in a total amount of at least 10 wt.%, more preferably 19 to 22 wt.%, more preferably the further diluent agent is microcrystalline cellulose or a combination of microcrystalline cellulose and xylitol. Furthermore, the intragranular composition comprises one, two or more disintegrant agents as set out above in a total amount of at least 1 wt.%, more preferably 2 wt.%, more preferably the disintegrant agent is sodium starch glycolate or croscarmellose sodium, more preferably sodium starch glycolate. In addition the extragranular composition comprises in addition to the reducing sugar content as diluent, one or more disintegrant agents, preferably in an amount of at least at least 3 wt.% or more, preferably 6 to 12 wt.%, more preferably the disintegrant agent is sodium starch glycolate or croscarmellose sodium or a mixture of sodium starch glycolate and croscarmellose sodium. Optionally the extragranular phase comprises one or more further binder agents, preferably in an amount of at least 0.5 wt.% or more, preferably 2 to 4 wt.%, more preferably povidone and optionally crospovidone. In addition the extragranular composition comprises at least one lubricant, preferably in an amount of at least 0.5 wt.% or more, preferably 0.9 to 1.2 wt.%, more preferably the lubricant is magnesium stearate. Furthermore the inventive tablet composition is film coated comprising suitable coating agents, preferably in an amount of least 2 wt.%, preferably 3 to 5 wt.%. The respective weight amounts as set out before respectively are based on the total weight of the inventive tablet composition. According to a furthermore preferred embodiment, the total weight of the inventive tablet composition is 363 mg.

According to one embodiment of the present invention where the inventive tablet composition comprises eltrombopag olamine in the extragranular composition, the intragranular composition comprises the reducing sugar, preferably lactose, more preferably lactose monohydrate, and a binder agent, preferably polyvinyl pyrrolidone, whereas the extragranular composition comprises in addition to a therapeutically active amount of eltrombopag olamine a diluent agent, preferably microcrystalline cellulose, and optionally one or more of the following pharmaceutically acceptable excipient agents, such as a binder agent, preferably polyvinyl pyrrolidone, a disintegrants agent, preferably sodium starch glycolate and a lubrication agent, preferably magnesium stearate. The resulting inventive tablet composition is preferably film coated with a suitable coating as disclosed above.

According to another embodiment of all aspects of the present invention as described herein before, the inventive pharmaceutical tablet composition additionally comprises or consists of a therapeutically effective amount of one or more additional active ingredients. The one or more additional active ingredients may be comprised in the intragranular composition or - in case of prevention of interaction between the active ingredients and eltrombopag olamine - in composition not comprising the eltrombopag olamine. Alternatively, the one or more additional active ingredients are simultaneously or sequentially co-administered in a separate pharmaceutical formulation. In a preferred embodiment, the one or more additional active ingredients are selected from constituents which are effective in the treatment of prophylaxis of thrombocytopenia, preferably adult chronic immune (idiopathic) thrombocytopenic purpura (ITP), acquired severe aplastic anaemia (SAA) and/or chronic hepatitis C virus (HCV) infection.

Accordingly, one or more additional active ingredients are selected from the group of corticosteroids, lithium carbonate, folate, rituximab, and immunoglobuline, in particular anti-Rh (D) immunoglobuline generally used to treat thrombocytopenia, in particular chronic immune (idiopathic) thrombocytopenic purpura (ITP).

Additionally or alternatively one or more additional active ingredients are selected from the group of anti-infective agents, such as antibiotics and antifungal agents, immunosuppressive agents, such as anti-thymocyte globulin (ATG) and cyclosporine-A (CSA) and cytokine agents, such as granulocyte colony-stimulating factor (G-CSF), granulocyte-macrophage colony-stimulating factor (GM-CSF), generally used to treat acquired severe aplastic anaemia (SAA).

Furthermore, one or more additional active ingredients are selected from the group of antiviral agents, such as daclatasvir, elbasvir, grazoprevir, ledipasvir, sofosbuvir, ombitasvir, paritaprevir, ritonavir, dasabuvir, simeprevir, sofosbuvir, velpatasvir, ribavirin, peginterferon alfa-2a or peginterferon alfa-2b generally used to treat chronic hepatitis C virus (HCV) infection.

The properties or the physical and chemical stability of the inventive pharmaceutical tablet composition may be tested in conventional manner, e.g. by measurement of appearance, hardness (or resistance to crushing), disintegration time, dissolution, friability, water content, assay for eltrombopag olamine and/or its degradation products (related substances), and/or uniformity of dosage units or mass after storage at controlled storage conditions; e.g. at intermediate and/or accelerated conditions according to ICH guideline Q1A(R2) (i.e. at 25 °C / 60 % relative humidity (RH) and/or at 40 °C / 75 % RH). These tests shall be performed according to applicable pharmaceutical regulatory standards as described e.g. in ICH or EMA guidelines and/or the European Pharmacopeia (EP).

At least some of these attributes, i.e. properties or physical and chemical stability, preferably most of these attributes and most preferably all of these attributes of the inventive pharmaceutical tablet composition are stable over time and different controlled storage conditions. In a preferred embodiment the dissolution (profile) of the inventive pharmaceutical tablet composition according to the present invention, e.g. a tablet or film-coated tablet, is stable over at least 6 months when stored preferably in Alu-Alu blisters at intermediate or long-term storage conditions, i.e. 25 °C / 60 % RH or 40 °C / 75 % RH. More preferably, dissolution and further additional attributes such as, e.g., assay, related substances or uniformity of dosage units or mass are also stable after storage over at least 6 months when stored at intermediate or long-term storage conditions. The term "stable" in the context of the present invention means that a measured value falls within range of specified values determined in accordance with a respective applicable regulatory guideline, e.g. the European Pharmacopeia.

The inventive pharmaceutical tablet composition, preferably film-coated tablet, may vary in shape and be, e.g., round, oval, oblong, cylindrical, caplet shaped or any other suitable shape, preferably it is round or caplet shaped. Furthermore, the inventive pharmaceutical tablet composition comprises means for dividing the tablet, such as scores or engravings.

The inventive pharmaceutical tablet composition, preferably film-coated tablet, has a diameter ranging between 3.5 and 15 mm and most preferably between 5.5 and 7.0 mm for round tablets and the dimension of a caplet is between 9.0 x 3.0 mm and 17.0 x 7.5 mm, preferably it is 12.7 x 5.9 mm. Thickness is ranging from 2.0 to 5.0 mm, preferably between 2.5 and 4.6 mm.

The inventive pharmaceutical tablet composition according to all aspects has a suitable hardness (or resistance to crushing), preferably the film-coated tablets may have a hardness (or resistance to crushing) of at least 40 N, preferably from 50 N to 130 N, more preferably 85 N to 110 N.

Furthermore, the inventive pharmaceutical tablet composition according to all aspects has a suitable disintegration time, preferably the film-coated tablets may have a disintegration time of at least 2 minutes, preferably in the range of 2 to 6 minutes.

Furthermore inventive pharmaceutical tablet composition, preferably film-coated tablet, may be colored and/or marked so as to impart an individual appearance and to make them instantly recognizable. The use of dyes can serve to enhance the appearance as well as to identify the inventive pharmaceutical composition. Dyes suitable for use in pharmacy typically include carotenoids, iron oxides or chlorophyll. The inventive pharmaceutical tablet composition, preferably a film-coated tablet, may be marked using an imprint code.

The inventive pharmaceutical composition may be packed in any conventional packaging known in the art, for example blisters, polypropylene or polyethylene (e.g. HDPE, high density polyethylene) containers or glass bottles. Preferably the inventive tablet composition is preferably packed in a blister known in the art, e.g. sealed aluminum blister (Alu-Alu), Aluminum / Aluminum peel-push blisters or PVC/PE/PVDC/aluminum-blisters.

According to the third aspect of the present invention the inventive process of production of a pharmaceutical tablet comprising eltrombopag olamine as active ingredient and one or more pharmaceutically acceptable excipients including one or more reducing sugars is characterized in that the process comprises or consists of the following steps:
either
a. mixing a therapeutically effective amount of eltrombopag olamine and one or more pharmaceutically acceptable excipients,
b. wet granulation of components mixed in step a) with a suitable granulation fluid, preferably water or an aqueous solution of a binder, to form an intragranular composition,
c. mixing one or more reducing sugars, preferably selected from the group consisting of lactose, maltose, glucose, arabinose, and fructose, preferably lactose and optionally further one or more pharmaceutically acceptable excipients to form an extragranular composition,
d. mixing the extragranular composition of step c) with the intragranular composition of step b) to form a tablet composition, and
e. compressing the tablet composition of step d) to form the pharmaceutical tablet composition comprising eltrombopag olamine as active ingredient in the intragranular phase and one or more reducing sugars in the extragranular phase,
   or
f. mixing one or more reducing sugars, preferably selected from the group consisting of lactose, maltose, glucose, arabinose, and fructose, preferably lactose and optionally further one or more pharmaceutically acceptable excipients,
g. wet granulation of components mixed in step f) with a suitable granulation fluid, preferably water or an aqueous solution of a binder, to form an intragranular composition,
h. mixing a therapeutically effective amount of eltrombopag olamine and one or more pharmaceutically acceptable excipients to form an extragranular composition,
i. mixing the extragranular composition of step h) with the intragranular composition of step g) to form a tablet composition, and
j. compressing the tablet composition of step i) to form the pharmaceutical tablet composition comprising eltrombopag olamine as active ingredient in the extragranular phase and one or more reducing sugars in the intragranular phase.

The preferred embodiments disclosed with respect to the inventive pharmaceutical tablet composition also apply in combination or separately with respect to the second aspect of the present invention.

Preferably the provided constituents, such as eltrombopag olamine, reducing sugars, disintegrants, binders, diluents and coating agents are sifted prior to use, preferably they are sifted through a # 35 mesh (Tyler Equivalent) screen, which corresponds to a screen with an opening of 0.420 mm in diameter.

According to a preferred embodiment the provided constituents in step a) or in step f) are loaded into a high shear mixer granulator or an equivalent granulator and preferably mixed for 10, more preferably 5 minutes with impeller at preferably slow speed.

Subsequently, the suitable granulation fluid, preferably water or an aqueous solution of a binder, is added to the mixture of step a) or step f) in the high shear mixer granulator and the impeller is preferably used at low speed.

According to a preferred embodiment the granules obtained in step c) or in step h) after wet granulation are subsequently dried at suitable conditions.

In a further preferred embodiment the granules obtained in step c) or in step h) preferably dried are subsequently milled, preferably using a Quadro^{®} Comil equipped with a suitable screen.

Subsequently, the (milled) granules forming the intragranular phase and sifted and admixed extragranular constituents are loaded into a blender and mixed preferably for 10 minutes according to step d) or step i). In case of using a lubricant, such as magnesium stearate, this constituent is preferably subsequently added to the blender and mixed preferably for further 5 minutes in step d) or step i).

Subsequently, the preferably lubricated mixture of step d) or step i) is compressed into the inventive pharmaceutical tablet composition preferably on a rotary tablet compression machine using suitable tooling and parameters in step e) or in step j).

The resulting inventive pharmaceutical tablet composition of step e) or step j) is furthermore preferably film coated in a subsequent step using one or more pharmaceutically acceptable excipients as suitable coating agent. The degradation of eltrombopag olamine may thereby be further reduced. Preferably the suitable coating agent comprises one or more polymers, optionally one or more plasticizers, and optionally one or more pigments. The preferred coating agents as disclosed with respect to the inventive pharmaceutical tablet composition can also be used with respect to the second aspect of the present invention.

As set out in the Example Section of the present application (see Part E, tables 1 and 2), the amount of impurities measured for the inventive pharmaceutical tablet compositions of the first and third aspect initially after production is below detection limit (BLD). When storing the inventive pharmaceutical tablet compositions under accelerated conditions at open exposure on a petri dish, the amount of impurities is still below detection limit for 40 % relative humidity (RH) and 50°C, 60°C and 70°C. When increasing the relative humidity to 75 % the impurities are detectable at a very low level for 50°C and an increased level for 75°C (see table 1). When using the regulatory accelerated stability assessment standards for Europe, the inventive pharmaceutical tablet compositions are sealed in Alu-Alu blisters and the amount of impurities is still below detection limit both after one or two months. Accordingly, the inventive pharmaceutical tablet compositions of the first and third aspect show a decreased degradation of eltrombopag olamine as active ingredient in comparison to the lactose composition of the prior art (see Kapsi declaration), where the composition already initially after production comprises 1.5 % total impurities. The reduced degradation of eltrombopag olamine may be due to the use of the inventive combination of the lactose with polyvinyl pyrrolidone and/or may be due to the inventive production process according to which the eltrombopag olamine constituent is formulated with polyvinyl pyrrolidone in one composition and the lactose is added together with further polyvinyl pyrrolidone in the other composition.

According to a fourth aspect of the present invention the inventive pharmaceutical tablet composition comprising eltrombopag olamine as active ingredient and one or more pharmaceutically acceptable excipients including one or more reducing sugars, characterized in that the composition comprising or consisting of
either
a) eltrombopag olamine in a therapeutically effective amount and one or more pharmaceutically acceptable excipients in an intragranular composition, and
b) one or more reducing sugars, preferably selected from the group consisting of lactose, maltose, glucose, arabinose, and fructose and optionally further one or more pharmaceutically acceptable excipients in an extragranular composition
   or
c) one or more reducing sugars, preferably selected from the group consisting of lactose, maltose, glucose, arabinose, and fructose and optionally further one or more pharmaceutically acceptable excipients in an intragranular composition and
d) eltrombopag olamine in a therapeutically effective amount and one or more pharmaceutically acceptable excipients in an extragranular composition.
is obtainable by use of the inventive production process.

According to a fifth aspect of the present invention the use the inventive pharmaceutical tablet composition is claimed, wherein the pharmaceutical tablet composition is used in the manufacture of a medicament for the treatment or prophylaxis of immune (idiopathic) thrombocytopenic purpura (ITP), thrombocytopenia and/or acquired severe aplastic anaemia (SAA).

According to a sixth aspect of the present invention the method of treatment or prophylaxis of immune (idiopathic) thrombocytopenic purpura (ITP), thrombocytopenia and/or acquired severe aplastic anaemia (SAA), comprises administering to a patient in need an inventive pharmaceutical tablet composition. The present invention is explained further with the aid of the following non-limiting examples, illustrating the parameters of and compositions employed within the present invention. Unless stated otherwise, all data, in particular percentages, parts and ratios are by weight.

According to the present invention the individual features of the exemplary embodiments of the inventive pharmaceutical tablet composition and the inventive production process as disclosed in the detailed description or claims of the present application can respectively be separately combined with features of the further exemplary embodiments herein below.

### Examples:

The eltrombopag olamine constituent used in the following example formulations F1 to 38 exhibits polymorphic form I.

### Part A: General Procedure for the wet granulation compression (eltrombopag olamine intragranular / lactose extragranular)

### Stage A: (Dry Mix)

1. Sifted Eltrombopag olamine and intragranular excipients through #35 mesh.
2. Sifted materials of step-1 loaded into high shear mixer granulator and mixed for 5 minutes with impeller at slow speed.

### Stage B: (Granulation)

3. Step-2 material was granulated with a suitable binder solution, e.g., water or an aqueous solution such as aqueous povidone solution, at impeller slow speed.
4. The granules of step-3 material were dried in a suitable equipment to get required loss on drying.
5. The dried granules of step-4 were milled using Quadro^{®} Comil equipped with suitable screen.

### Stage C: (Blending)

6. Extragranular materials were sifted through #35 mesh.
7. Milled granules of step 5 and sifted extragranular materials of step 6 were loaded into the blender and mixed for 10 min.

### Stage D: (Lubrication)

8. Magnesium stearate sifted through #35 mesh and added to blend mixture of step 7 and lubricated for 5 minutes.
9. The blend mixture of step 8 was compressed into tablets on rotary tablet compression machine using suitable tooling and parameters.

### Stage E: Film-Coating

10. Core tablets of step 9 were coated in coating pan using corresponding Opadry^{®} suspension.

### Part B: Process of production of inventive pharmaceutical tablet compositions (eltrombopag olamine intragranular / lactose extragranular)

The following inventive pharmaceutical tablet compositions F1-F37 are produced in accordance with the general procedure as set out in Part A above:
**Example F1:** inventive pharmaceutical film-coated tablet composition comprising lactose monohydrate, microcrystalline cellulose, sodium starch glycolate and magnesium stearate in extragranular phase and eltrombopag olamine, microcrystalline cellulose and polyvinyl pyrrolidone (povidone) in intragranular phase

| **Ingredients** | **F1 (mg/unit)** |
|---|---|
| **Stage-A: (Dry Mix)** | |
| Eltrombopag Olamine, *In-house* | 96.902 |
| Cellulose, Microcrystalline *Ph.Eur.* | 82.698 |
| Polyvinyl pyrrolidone, Povidone *Ph.Eur.* (Plasdone K29/32) | 9.600 |
| **Stage-B: (Binder Solution / Granulation)** | |
| Water, purified *Ph.Eur.* | q.s. |
| **Stage-C (Blending)** | |
| Cellulose, Microcrystalline *Ph.Eur.* | 79.300 |
| Microcelac^{®} (Lactose monohydrate in an amount of 75 wt. % and microcrystalline cellulose 25 wt. % respectively based on the total weight of Microcelac^{®}) *In-house* | 48.000 |
| Sodium starch glycolate Type-A *Ph.Eur.* (Explotab) | 28.000 |
| **Stage-D (Blending & Lubrication)** | |
| Magnesium stearate *Ph.Eur.* | 3.500 |
| **Core Tablet weight (mg)** | **348.000** |
| **Stage-E (Film-coating)** | |
| Opadry^{®} Pink *In-house* | 15.000 |
| Water, Purified *Ph.Eur.* | q.s. |
| **Film-coated tablet weight (mg)** | 363.000 |

**Example F2:** inventive pharmaceutical film-coated tablet composition comprising lactose monohydrate, microcrystalline cellulose, sodium starch glycolate and magnesium stearate in extragranular phase and eltrombopag olamine, microcrystalline cellulose and polyvinyl pyrrolidone (povidone) in intragranular phase

| **Ingredients** | **F2 (mg/unit)** |
|---|---|
| **Stage-A: (Dry Mix)** | |
| Eltrombopag Olamine, *In-house* | 96.902 |
| Cellulose, Microcrystalline *Ph.Eur.* | 118.698 |
| Polyvinyl pyrrolidone, Povidone *Ph.Eur.* (Plasdone K29/32) | 9.600 |
| **Stage-B: (Binder Solution / Granulation)** | |
| Water, purified *Ph.Eur.* | q.s. |
| **Stage-C (Blending)** | |
| Cellulose, Microcrystalline *Ph.Eur.* | 79.300 |
| Lactose monohydrate *Ph.Eur.* (Tablettose 100) | 12.000 |
| Sodium starch glycolate Type-A *Ph.Eur.* (Explotab) | 28.000 |
| **Stage-D (Blending & Lubrication)** | |
| Magnesium stearate *Ph.Eur.* | 3.500 |
| **Core Tablet weight (mg)** | **348.000** |
| **Stage-E (Film-coating)** | |
| Opadry^{®} Pink *In-house* | 15.000 |
| Water, Purified *Ph.Eur.* | q.s. |
| **Film-coated tablet weight (mg)** | **363.000** |

**Example F3:** inventive pharmaceutical film-coated tablet composition comprising lactose monohydrate, microcrystalline cellulose, polyvinyl pyrrolidone (povidone), crosslinked insoluble polyvinylpyrrolidone (crospovidone), sodium starch glycolate and magnesium stearate in extragranular phase and eltrombopag olamine, microcrystalline cellulose and polyvinyl pyrrolidone (povidone) in intragranular phase

| **Ingredients** | **F3 (mg/unit)** |
|---|---|
| **Stage-A: (Dry Mix)** | |
| Eltrombopag Olamine, *In-house* | 96.902 |
| Cellulose, Microcrystalline *Ph.Eur.* | 91.698 |
| Polyvinyl pyrrolidone, Povidone *Ph.Eur.* (Plasdone K29/32) | 9.600 |
| **Stage-B: (Binder Solution / Granulation)** | |
| Water, purified *Ph.Eur.* | q.s. |
| **Stage-C (Blending)** | |
| Cellulose, Microcrystalline *Ph.Eur.* | 79.300 |
| Ludipress^{®} (Lactose monohydrate in an amount of 93 wt. %, polyvinyl pyrrolidone, Povidone, Kollidon^{®} 30 in an amount of 3.5 wt. % and crosslinked insoluble polyvinylpyrrolidone, Crospovidone, Kollidon^{®} CL 3.5 wt. % respectively based on the total weight of Ludipress^{®}) | 39.000 |
| Sodium starch glycolate Type-A *Ph.Eur.* (Explotab) | 28.000 |
| **Stage-D (Blending & Lubrication)** | |
| Magnesium stearate *Ph.Eur.* | 3.500 |
| **Core Tablet weight (mg)** | **348.000** |
| **Stage-E (Film-coating)** | |
| Opadry^{®} Pink *In-house* | 15.000 |
| Water, Purified *Ph.Eur.* | q.s. |
| **Film-coated tablet weight (mg)** | **363.000** |

**Example F4:** inventive pharmaceutical film-coated tablet composition comprising lactose monohydrate, microcrystalline cellulose, sodium starch glycolate and magnesium stearate in extragranular phase and eltrombopag olamine, microcrystalline cellulose and polyvinyl pyrrolidone (povidone) in intragranular phase

| **Ingredients** | **F4 (mg/unit)** |
|---|---|
| **Stage-A: (Dry Mix)** | |
| Eltrombopag Olamine, *In-house* | 96.902 |
| Cellulose, Microcrystalline *Ph.Eur.* | 94.698 |
| Polyvinyl pyrrolidone, Povidone *Ph.Eur.* (Plasdone K29/32) | 9.600 |
| **Stage-B: (Binder Solution / Granulation)** | |
| Water, purified *Ph.Eur.* | q.s. |
| **Stage-C (Blending)** | |
| Cellulose, Microcrystalline *Ph.Eur.* | 79.300 |
| Lactose monohydrate *Ph.Eur.* (Tablettose 100) | 36.000 |
| Sodium starch glycolate Type-A *Ph.Eur.* (Explotab) | 28.000 |
| **Stage-D (Blending & Lubrication)** | |
| Magnesium stearate *Ph.Eur.* | 3.500 |
| **Core Tablet weight (mg)** | **348.000** |
| **Stage-E (Film-coating)** | |
| Opadry^{®} Pink *In-house* | 15.000 |
| Water, Purified *Ph.Eur.* | q.s. |
| **Film-coated tablet weight (mg)** | **363.000** |

**Example F5:** inventive pharmaceutical film-coated tablet composition comprising lactose monohydrate, xylitol, sodium starch glycolate and magnesium stearate in extragranular phase and eltrombopag olamine, microcrystalline cellulose and polyvinyl pyrrolidone (povidone) in intragranular phase

| **Ingredients** | **F5 (mg/unit)** |
|---|---|
| **Stage-A: (Dry Mix)** | |
| Eltrombopag Olamine, *In-house* | 96.902 |
| Cellulose, Microcrystalline *Ph.Eur.* | 73.000 |
| Polyvinyl pyrrolidone, Povidone *Ph.Eur.* (Plasdone K29/32) | 9.600 |
| **Stage-B: (Binder Solution / Granulation)** | |
| Water, purified *Ph.Eur.* | q.s. |
| **Stage-C (Blending)** | |
| Xylitol *Ph.Eur.* | 92.998 |
| Lactose monohydrate *Ph.Eur.* (Tablettose 100) | 44.000 |
| Sodium starch glycolate Type-A *Ph.Eur.* (Explotab) | 28.000 |
| **Stage-D (Blending & Lubrication)** | |
| Magnesium stearate *Ph.Eur.* | 3.500 |
| **Core Tablet weight (mg)** | **348.000** |
| **Stage-E (Film-coating)** | |
| Opadry^{®} Brown *In-house* | 15.000 |
| Water, Purified *Ph.Eur.* | q.s. |
| **Film-coated tablet weight (mg)** | **363.000** |

**Example F6:** inventive pharmaceutical film-coated tablet composition comprising lactose monohydrate, xylitol, sodium starch glycolate and magnesium stearate in extragranular phase and eltrombopag olamine, microcrystalline cellulose and polyvinyl pyrrolidone (povidone) in intragranular phase

| **Ingredients** | **F6 (mg/unit)** |
|---|---|
| **Stage-A: (Dry Mix)** | |
| Eltrombopag Olamine, *In-house* | 96.902 |
| Cellulose, Microcrystalline *Ph.Eur.* | 73.000 |
| Polyvinyl pyrrolidone, Povidone *Ph.Eur.* (Plasdone K29/32) | 9.600 |
| **Stage-B: (Binder Solution / Granulation)** | |
| Water, purified *Ph.Eur.* | q.s. |
| **Stage-C (Blending)** | |
| Xylitol *Ph.Eur.* | 81.998 |
| Lactose monohydrate *Ph.Eur.* (Tablettose 100) | 55.000 |
| Sodium starch glycolate Type-A *Ph.Eur.* (Explotab) | 28.000 |
| **Stage-D (Blending & Lubrication)** | |
| Magnesium stearate *Ph.Eur.* | 3.500 |
| **Core Tablet weight (mg)** | **348.000** |
| **Stage-E (Film-coating)** | |
| Opadry^{®} Brown *In-house* | 15.000 |
| Water, Purified *Ph.Eur.* | q.s. |
| **Film-coated tablet weight (mg)** | **363.000** |

**Example F7:** inventive pharmaceutical film-coated tablet composition comprising lactose monohydrate, xylitol, sodium starch glycolate and magnesium stearate in extragranular phase and eltrombopag olamine, microcrystalline cellulose and polyvinyl pyrrolidone (povidone) in intragranular phase

| **Ingredients** | **F7 (mg/unit)** |
|---|---|
| **Stage-A: (Dry Mix)** | |
| Eltrombopag Olamine, *In-house* | 96.902 |
| Cellulose, Microcrystalline *Ph.Eur.* | 73.000 |
| Polyvinyl pyrrolidone, Povidone *Ph.Eur.* (Plasdone K29/32) | 9.600 |
| **Stage-B: (Binder Solution / Granulation)** | |
| Water, purified *Ph.Eur.* | q.s. |
| **Stage-C (Blending)** | |
| Xylitol *Ph.Eur.* | 9.998 |
| Lactose monohydrate *Ph.Eur.* (Tablettose 100) | 127.000 |
| Sodium starch glycolate Type-A *Ph.Eur.* (Explotab) | 28.000 |
| **Stage-D (Blending & Lubrication)** | |
| Magnesium stearate *Ph.Eur.* | 3.500 |
| **Core Tablet weight (mg)** | 348.000 |
| **Stage-E (Film-coating)** | |
| Opadry^{®} Brown *In-house* | 15.000 |
| Water, Purified *Ph.Eur.* | q.s. |
| **Film-coated tablet weight (mg)** | **363.000** |

**Examples F8 and F9:** inventive pharmaceutical film-coated tablet compositions comprising co-processed lactose comprising lactose monohydrate, polyvinyl pyrrolidone (povidone) and crosslinked insoluble polyvinyl pyrrolidone (crospovidone), sodium starch glycolate and magnesium stearate in extragranular phase and eltrombopag olamine, microcrystalline cellulose and polyvinyl pyrrolidone (povidone) in intragranular phase

| **Ingredients** | **F8 (mg/unit)** | **F9 (mg/unit)** |
|---|---|---|
| **Stage-A: (Dry Mix)** | | |
| Eltrombopag Olamine, *In-house* | 96.902 | 95.703 |
| Cellulose, Microcrystalline *Ph.Eur.* | 73.000 | 72.365 |
| Polyvinyl pyrrolidone, Povidone *Ph.Eur.* (Plasdone K29/32) | 9.600 | 9.600 |
| **Stage-B: (Binder Solution / Granulation)** | | |
| Water, purified *Ph.Eur.* | q.s. | q.s. |
| **Stage-C (Blending)** | | |
| Ludipress^{®} (Lactose monohydrate in an amount of 93 wt. %, polyvinyl pyrrolidone, Povidone, Kollidon^{®} 30 in an amount of 3.5 wt. % and crosslinked insoluble polyvinylpyrrolidone, Crospovidone, Kollidon^{®} CL 3.5 wt. % respectively based on the total weight of Ludipress^{®}) | 137.000 | 138.832 |
| Sodium starch glycolate Type-A *Ph.Eur.* (Explotab) | 28.000 | 28.000 |
| **Stage-D (Blending & Lubrication)** | | |
| Magnesium stearate *Ph.Eur.* | 3.500 | 3.500 |
| **Core Tablet weight (mg)** | **348.000** | **348.000** |
| **Stage-E (Film-coating)** | | |
| Opadry^{®} Brown *In-house* | 15.000 | 15.000 |
| Water, Purified *Ph.Eur.* | q.s. | q.s. |
| **Film-coated tablet weight (mg)** | **363.000** | **363.000** |

**Examples F10 and F11:** inventive pharmaceutical film-coated tablet compositions comprising lactose monohydrate, polyvinyl pyrrolidone (povidone), sodium starch glycolate and magnesium stearate in extragranular phase and eltrombopag olamine, microcrystalline cellulose and polyvinyl pyrrolidone (povidone) in intragranular phase

| **Ingredients** | **F10 (mg/unit)** | **F11 (mg/unit)** |
|---|---|---|
| **Stage-A: (Dry Mix)** | | |
| Eltrombopag Olamine, *In-house* | 96.902 | 95.703 |
| Cellulose, Microcrystalline *Ph.Eur.* | 73.000 | 72.365 |
| Polyvinyl pyrrolidone, Povidone *Ph.Eur.* (Plasdone K29/32) | 9.600 | 9.600 |
| **Stage-B: (Binder Solution / Granulation)** | | |
| Water, purified *Ph.Eur.* | q.s. | q.s. |
| **Stage-C (Blending)** | | |
| Lactose monohydrate *Ph.Eur.* (Tablettose 100) | 127.000 | 128.832 |
| Polyvinyl pyrrolidone, Povidone *Ph.Eur.* (Plasdone K29/32) | 9.998 | 10.000 |
| Sodium starch glycolate Type-A *Ph.Eur.* (Explotab) | 28.000 | 28.000 |
| **Stage-D (Blending & Lubrication)** | | |
| Magnesium stearate *Ph.Eur.* | 3.500 | 3.500 |
| **Core Tablet weight (mg)** | **348.000** | **348.000** |
| **Stage-E (Film-coating)** | | |
| Opadry^{®} Brown *In-house* | 15.000 | 15.000 |
| Water, Purified *Ph.Eur.* | q.s. | q.s. |
| **Film-coated tablet weight (mg)** | **363.000** | **363.000** |

**Example F12:** inventive pharmaceutical film-coated tablet composition comprising lactose monohydrate, microcrystalline cellulose, sodium starch glycolate and magnesium stearate in extragranular phase and eltrombopag olamine, microcrystalline cellulose, xylitol and polyvinyl pyrrolidone (povidone) in intragranular phase

| **Ingredients** | **F12 (mg/unit)** |
|---|---|
| **Stage-A: (Dry Mix)** | |
| Eltrombopag Olamine, *In-house* | 96.251 |
| Cellulose, Microcrystalline *Ph.Eur.* (Comprecel M 102 D+) | 45.000 |
| Xylitol | 80.000 |
| Polyvinyl pyrrolidone, Povidone *Ph.Eur.* (Plasdone K29/32) | 10.000 |
| **Stage-B: (Binder Solution / Granulation)** | |
| Water, purified *Ph.Eur.* | q.s. |
| **Stage-C (Blending)** | |
| Cellulose, Microcrystalline *Ph.Eur.* (Comprecel M 102 D+) | 27.900 |
| Lactose monohydrate *Ph.Eur.* | 57.349 |
| Sodium starch glycolate Type-A *Ph.Eur.* (Explotab) | 28.000 |
| **Stage-D (Blending & Lubrication)** | |
| Magnesium stearate *Ph.Eur.* | 3.500 |
| **Core Tablet weight (mg)** | **348.000** |
| **Stage-E (Film-coating)** | |
| Opadry^{®} Brown *In-house* | 15.000 |
| Water, Purified *Ph.Eur.* | q.s. |
| **Film-coated tablet weight (mg)** | **363.000** |

**Example F13:** inventive pharmaceutical film-coated tablet composition comprising anhydrous lactose, sodium starch glycolate and magnesium stearate in extragranular phase and eltrombopag olamine, microcrystalline cellulose, sodium starch glycolate and polyvinyl pyrrolidone (povidone) in intragranular phase

| **Ingredients** | **F13(mg/unit)** |
|---|---|
| **Stage-A: (Dry Mix)** | |
| Eltrombopag Olamine, *In-house* | 96.338 |
| Cellulose, Microcrystalline *Ph.Eur.* | 73.000 |
| Polyvinyl pyrrolidone, Povidone *Ph.Eur.* (Plasdone K29/32) | 20.000 |
| Sodium starch glycolate Type-A *Ph.Eur.* (Explotab) | 14.000 |
| **Stage-B: (Binder Solution / Granulation)** | |
| Water, purified *Ph.Eur.* | q.s. |
| **Stage-C (Blending)** | |
| Lactose anhydrous *Ph.Eur.* (Supertab 21 AN) | 127.162 |
| Sodium starch glycolate Type-A *Ph.Eur.* (Explotab) | 14.000 |
| **Stage-D (Blending & Lubrication)** | |
| Magnesium stearate *Ph.Eur.* | 3.500 |
| **Core Tablet weight (mg)** | **348.000** |
| **Stage-E (Film-coating)** | |
| Opadry^{®} Brown *In-house* | 15.000 |
| Water, Purified *Ph.Eur.* | q.s. |
| **Film-coated tablet weight (mg)** | **363.000** |

**Example F14:** inventive pharmaceutical film-coated tablet composition comprising lactose monohydrate, microcrystalline cellulose, sodium starch glycolate and magnesium stearate in extragranular phase and eltrombopag olamine, microcrystalline cellulose, and polyvinyl pyrrolidone (povidone) in intragranular phase

| **Ingredients** | **F14 (mg/unit)** |
|---|---|
| **Stage-A: (Dry Mix)** | |
| Eltrombopag Olamine, *In-house* | 96.251 |
| Cellulose, Microcrystalline *Ph.Eur.* (Comprecel M 102 D+) | 18.269 |
| Sodium starch glycolate Type-A *Ph.Eur.* (Explotab) | 7.000 |
| **Stage-B: (Binder Solution / Granulation)** | |
| Polyvinyl pyrrolidone, Povidone *Ph.Eur.* (Plasdone K29/32) | 10.000 |
| Water, purified *Ph.Eur.* | q.s. |
| **Stage-C (Blending)** | |
| Cellulose, Microcrystalline *Ph.Eur.* (Comprecel M 102 D+) | 54.000 |
| Lactose monohydrate *Ph.Eur.* (Flowlac 90) | 137.980 |
| Sodium starch glycolate Type-A *Ph.Eur.* (Explotab) | 21.000 |
| **Stage-D (Blending & Lubrication)** | |
| Magnesium stearate *Ph.Eur.* | 3.500 |
| **Core Tablet weight (mg)** | **348.000** |
| **Stage-E (Film-coating)** | |
| Opadry^{®} Brown *In-house* | 15.000 |
| Water, Purified *Ph.Eur.* | q.s. |
| **Film-coated tablet weight (mg)** | **363.000** |

**Example F15:** inventive pharmaceutical film-coated tablet composition comprising lactose monohydrate, microcrystalline cellulose, croscarmellose sodium and magnesium stearate in extragranular phase and eltrombopag olamine, microcrystalline cellulose and polyvinyl pyrrolidone (povidone) in intragranular phase

| **Ingredients** | **F15(mg/unit)** |
|---|---|
| **Stage-A: (Dry Mix)** | |
| Eltrombopag Olamine, *In-house* | 96.251 |
| Cellulose, Microcrystalline *Ph.Eur.* (Comprecel M 102 D+) | 36.269 |
| **Stage-B: (Binder Solution / Granulation)** | |
| Polyvinyl pyrrolidone, Povidone *Ph.Eur.* (Plasdone K29/32) | 10.000 |
| Water, purified *Ph.Eur.* | q.s. |
| **Stage-C (Blending)** | |
| Cellulose, Microcrystalline *Ph.Eur.* (Comprecel M 102 D+) | 36.000 |
| Lactose monohydrate *Ph.Eur.* (Flowlac 90) | 137.980 |
| Croscarmellose sodium *Ph.Eur.* (Ac-Di-Sol) | 28.000 |
| **Stage-D (Blending & Lubrication)** | |
| Magnesium stearate *Ph.Eur.* | 3.500 |
| **Core Tablet weight (mg)** | **348.000** |
| **Stage-E (Film-coating)** | |
| Opadry^{®} Brown *In-house* | 15.000 |
| Water, Purified *Ph.Eur.* | q.s. |
| **Film-coated tablet weight (mg)** | **363.000** |

**Example F16:** inventive pharmaceutical film-coated tablet composition comprising lactose monohydrate, microcrystalline cellulose, crospovidone and magnesium stearate in extragranular phase and eltrombopag olamine, microcrystalline cellulose and polyvinyl pyrrolidone (povidone) in intragranular phase

| **Ingredients** | **F16 (mg/unit)** |
|---|---|
| **Stage-A: (Dry Mix)** | |
| Eltrombopag Olamine, *In-house* | 96.251 |
| Cellulose, Microcrystalline *Ph.Eur.* (Comprecel M 102 D+) | 36.269 |
| **Stage-B: (Binder Solution / Granulation)** | |
| Polyvinyl pyrrolidone, Povidone *Ph.Eur.* (Plasdone K29/32) | 10.000 |
| Water, purified *Ph.Eur.* | q.s. |
| **Stage-C (Blending)** | |
| Cellulose, Microcrystalline *Ph.Eur.* (Comprecel M 102 D+) | 36.000 |
| Lactose monohydrate *Ph.Eur.* (Flowlac 90) | 137.980 |
| Crospovidone *Ph.Eur.* | 28.000 |
| **Stage-D (Blending & Lubrication)** | |
| Magnesium stearate *Ph.Eur.* | 3.500 |
| **Core Tablet weight (mg)** | **348.000** |
| **Stage-E (Film-coating)** | |
| Opadry^{®} Brown *In-house* | 15.000 |
| Water, Purified *Ph.Eur.* | q.s. |
| **Film-coated tablet weight (mg)** | **363.000** |

**Examples F17 to F20:** inventive pharmaceutical film-coated tablet composition comprising lactose monohydrate, polyvinyl pyrrolidone (povidone), sodium starch glycolate and magnesium stearate in extragranular phase and 16 mg, 31.1 mg or 63.2 mg eltrombopag olamine (equivalent to 12.5 mg, 25 mg or 50 mg eltrombopag) in combination with microcrystalline cellulose and povidone in intragranular phase

| **Ingredients** | **F17** | **F18** | **F19** | **F20** |
|---|---|---|---|---|
| **Stage-A: (Dry Mix)** | **(mg/unit)** | **(mg/unit)** | **(mg/unit)** | **(mg/unit)** |
| Eltrombopag Olamine, *In-house* | 63.167 | 31.084 | 16.042 | 16.042 |
| Cellulose, Microcrystalline *Ph.Eur. (Comprecel M 102 D+)* | 73.000 | 73.00 | 73.00 | 36.500 |
| Povidone *Ph.Eur.* (Plasdone K29/32) | 9.600 | 9.60 | 9.60 | 4.800 |
| Water, Purified *Ph.Eur.* | q.s. | q.s. | q.s. | q.s. |
| **Stage-B (Blending)** | | | | |
| Lactose monohydrate *Ph.Eur.* (Tablettose 100) | 160.733 | 192.816 | 207.858 | 95.908 |
| Povidone *Ph.Eur.* (Plasdone K29/32) | 10.000 | 10.000 | 10.000 | 5.000 |
| Sodium starch glycolate Type-A *Ph.Eur.* (Explotab) | 28.000 | 28.000 | 28.000 | 14.000 |
| **Stage-C (Lubrication)** | | | | |
| Magnesium stearate *Ph.Eur.* | 3.500 | 3.500 | 3.500 | 1.750 |
| **Core Tablet weight (mg)** | **348.000** | **348.000** | **348.000** | **348.000** |
| **Stage-D (Film-coating)** | | | | |
| Opadry ^{®} Brown *In-house* | 15.000 | - | - | - |
| Opadry ^{®} White *In-house* | - | 15.000 | 15.000 | 7.500 |
| Water, Purified *Ph.Eur.* | q.s. | q.s. | q.s. | q.s. |
| **Film-coated tablet weight (mg)** | **363.000** | **363.000** | **363.000** | **363.000** |

**Examples F21 and F22:** inventive pharmaceutical film-coated tablet composition comprising Ludipress^{®} (93 wt.% lactose monohydrate, 3.5 wt.% polyvinyl pyrrolidone (Kollidon 30) and 3.5 wt.% crospovidone (Kollidon CL)) and sodium starch glycolate and magnesium stearate in extragranular phase and 16 mg eltrombopag olamine (equivalent to 12.5 mg eltrombopag) in combination with microcrystalline cellulose and povidone in intragranular phase

| **Ingredients** | **F21** | **F22** |
|---|---|---|
| **Stage-A: (Dry Mix)** | **(mg/unit)** | **(mg/unit)** |
| Eltrombopag Olamine, *In-house* | 16.042 | 16.042 |
| Cellulose, Microcrystalline *Ph.Eur.* (Comprecel M 102 D+) | 73.000 | 36.500 |
| Povidone *Ph.Eur.* (Plasdone K29/32) | 9.600 | 4.80 |
| Water, Purified *Ph.Eur.* | q.s. | q.s. |
| **Stage-B (Blending)** | | |
| Ludipress^{®} (93% Lactose monohydrate, 3.5% Povidone, and 3.5% Crospovidone) | 217.858 | 100.908 |
| Povidone *Ph.Eur.* (Plasdone K29/32) | - | - |
| Sodium starch glycolate Type-A *Ph.Eur.* (Explotab) | 28.000 | 14.000 |
| **Stage-C (Lubrication)** | | |
| Magnesium stearate *Ph.Eur.* | 3.500 | 1.750 |
| **Core Tablet weight (mg)** | **348.000** | **174.000** |
| **Stage-D (Film-coating)** | | |
| Opadry ^{®} White *In-house* | 15.000 | 7.500 |
| Water, Purified *Ph.Eur.* | q.s. | q.s. |
| **Film-coated tablet weight (mg)** | **363.000** | **181.500** |

**Examples F23 and F24:** inventive pharmaceutical film-coated tablet composition comprising lactose monohydrate, copovidone, sodium starch glycolate and magnesium stearate in extragranular phase and 16 mg eltrombopag olamine (equivalent to 12.5 mg eltrombopag) in combination with microcrystalline cellulose and copovidone in intragranular phase

| **Ingredients** | **F23** | **F24** |
|---|---|---|
| **Stage-A: (Dry Mix)** | **(mg/unit)** | **(mg/unit)** |
| Eltrombopag Olamine, *In-house* | 16.042 | 16.042 |
| Cellulose, Microcrystalline *Ph.Eur. (Comprecel M 102 D+)* | 73.000 | 36.500 |
| Copovidone *Ph.Eur.* (Plasdone S 630) | 9.600 | 4.800 |
| Water, Purified *Ph.Eur.* | q.s. | q.s. |
| **Stage-B (Blending)** | | |
| Lactose monohydrate *Ph.Eur.* (Tablettose 100) | 207.858 | 95.908 |
| Copovidone *Ph.Eur.* (Plasdone S 630) | 10.000 | 5.000 |
| Sodium starch glycolate Type-A *Ph.Eur.* (Explotab) | 28.000 | 14.000 |
| **Stage-C (Lubrication)** | | |
| Magnesium stearate *Ph.Eur.* | 3.500 | 1.750 |
| **Core Tablet weight (mg)** | **348.000** | **174.000** |
| **Stage-D (Film-coating)** | | |
| Opadry ^{®} White *In-house* | 15.000 | 7.500 |
| Water, Purified *Ph.Eur.* | q.s. | q.s. |
| **Film-coated tablet weight (mg)** | **363.000** | **181.500** |

**Examples F25 to F28:** inventive pharmaceutical film-coated tablet composition comprising lactose monohydrate, microcrystalline cellulose, croscarmellose sodium, sodium starch glycolate and magnesium stearate in extragranular phase and 16 mg, 31.9 mg, 63.2 mg or 95.7 mg eltrombopag olamine (equivalent to 12.5 mg, 25 mg, 50 mg or 75 mg eltrombopag) in combination with microcrystalline cellulose, polyvinyl pyrrolidone and sodium starch glycolate in intragranular phase

| **Ingredients** | **F25** | **F26** | **F27** | **F28** |
|---|---|---|---|---|
| **Stage-A: (Dry Mix)** | **(mg/unit)** | **(mg/unit)** | **(mg/unit)** | **(mg/unit)** |
| Eltrombopag Olamine, *In-house* | 95.703 | 63.802 | 31.901 | 15.951 |
| Cellulose, Microcrystalline *Ph. Eur. (Comprecel M 101 D*+*)* | 18.000 | 12.000 | 6.000 | 3.000 |
| Sodium starch glycolate Type-A *Ph.Eur.* (Explotab) | 7.000 | 4.670 | 2.333 | 1.167 |
| **Stage-B (Binder solution)** | | | | |
| Povidone *Ph.Eur.* (Plasdone K29/32) | 10.000 | 6.667 | 3.333 | 1.167 |
| Water, Purified *Ph.Eur.* | q.s. | q.s. | q.s. | q.s. |
| **Stage-C (Blending)** | | | | |
| Cellulose, Microcrystalline *Ph.Eur.* (Avicel PH 200) | 54.000 | 36.000 | 18.000 | 9.000 |
| Lactose monohydrate *Ph. Eur.* (Tablettose 80) | 126.797 | 84.530 | 42.267 | 21.133 |
| Croscarmellose sodium *Ph. Eur. (Ac-di-sol SD 711)* | 5.000 | 3.333 | 1.667 | 0.833 |
| Sodium starch glycolate Type-A *Ph.Eur.* (Explotab) | 28.000 | 18.667 | 9.333 | 4.667 |
| **Stage-D (Lubrication)** | | | | |
| Magnesium stearate *Ph.Eur.* | 3.5000 | 2.333 | 1.167 | 0.583 |
| **Core Tablet weight (mg)** | **348.000** | **232.000** | **116.000** | **58.000** |
| **Stage-E (Film-coating)** | | | | |
| Opadry ^{®} Brown 85F565229 *In-house* | 15.000 | - | - | - |
| Opadry ^{®} Brown 85F565230 *In-house* | - | 10.000 | - | - |
| Opadry ^{®} White *In-house* | - | - | 5.000 | 2.500 |
| Water, Purified *Ph.Eur.* | q.s. | q.s. | q.s. | q.s. |
| **Film-coated tablet weight (mg)** | **363.000** | **242.000** | **121.000** | **60.500** |

**Examples F29 to F32**: inventive pharmaceutical film-coated tablet composition comprising lactose monohydrate, microcrystalline cellulose, croscarmellose sodium, sodium starch glycolate and magnesium stearate in extragranular phase and 16 mg, 31.9 mg, or 63.2 mg eltrombopag olamine (equivalent to 12.5 mg, 25 mg, or 50 mg eltrombopag) in combination with microcrystalline cellulose, polyvinyl pyrrolidone (povidone) and sodium starch glycolate in intragranular phase

| **Ingredients** | **F29** | **F30** | **F31** |
|---|---|---|---|
| **Stage-A: (Dry Mix)** | **(mg/unit)** | **(mg/unit)** | **(mg/unit)** |
| Eltrombopag Olamine, *In-house* | 63.802 | 31.901 | 15.951 |
| Cellulose, Microcrystalline *Ph.Eur. (Comprecel M 101 D+)* | 12.000 | 6.000 | 3.000 |
| Sodium starch glycolate Type-A *Ph.Eur.* (Explotab) | 4.667 | 2.333 | 1.167 |
| **Stage-B (Binder solution)** | | | |
| Povidone *Ph.Eur.* (Plasdone K29/32) | 6.667 | 3.333 | 1.667 |
| Water, Purified *Ph.Eur.* | q.s. | q.s. | q.s. |
| **Stage-C (Blending)** | | | |
| Cellulose, Microcrystalline *Ph.Eur.* (Avicel PH 200) | 54.000 | 54.000 | 27.000 |
| Lactose monohydrate *Ph.Eur.* (Tablettose 80) | 170.365 | 213.932 | 106.966 |
| Croscarmellose sodium *Ph.Eur. (Ac-di-sol SD 711)* | 5.000 | 5.000 | 2.500 |
| Sodium starch glycolate Type-A *Ph.Eur.* (Explotab) | 28.000 | 28.000 | 14.000 |
| **Stage-D (Lubrication)** | | | |
| Magnesium stearate *Ph.Eur.* | 3.500 | 3.500 | 1.750 |
| **Core tablet weight (mg)** | **348.000** | **348.000** | **174.000** |
| **Stage-E (Film-coating)** | | | |
| Opadry ^{®} Brown *In-house* | 15.000 | - | - |
| Opadry ^{®} White *In-house* | - | 15.000 | 7.500 |
| Water, Purified *Ph.Eur.* | q.s. | q.s. | q.s. |
| **Film-coated tablet weight (mg)** | **363.000** | **363.000** | **181.500** |

**Example F32:** inventive pharmaceutical film-coated tablet composition comprising lactose monohydrate, mannitol, sodium starch glycolate and magnesium stearate in extragranular phase and 96.25 mg eltrombopag olamine (equivalent to 75 mg eltrombopag), microcrystalline cellulose, sodium starch glycolate, and polyvinyl pyrrolidone (povidone) in intragranular phase

| **Ingredients** | **F32** |
|---|---|
| **Stage-A: (Dry Mix)** | **(mg/unit)** |
| Eltrombopag Olamine, *In-house* | 96.251 |
| Cellulose, Microcrystalline *Ph.Eur.* (Comprecel M 102 D+) | 18.269 |
| Sodium starch glycolate Type-A *Ph.Eur.* (Explotab) | 7.000 |
| **Stage-B: (Binder Solution / Granulation)** | |
| Polyvinyl pyrrolidone, Povidone *Ph.Eur.* (Plasdone K29/32) | 10.000 |
| Water, purified *Ph.Eur.* | q.s. |
| **Stage-C (Blending)** | |
| Mannitol *Ph.Eur.* (Pearlitol SD 200) | 54.000 |
| Lactose monohydrate *Ph.Eur.* (Flowlac 90) | 134.980 |
| Sodium starch glycolate Type-A *Ph.Eur.* (Explotab) | 24.000 |
| **Stage-D (Blending & Lubrication)** | |
| Magnesium stearate *Ph.Eur.* | 3.500 |
| **Core Tablet weight (mg)** | **348.000** |
| **Stage-E (Film-coating)** | |
| Opadry^{®} Brown *In-house* | 15.000 |
| Water, Purified *Ph.Eur.* | q.s. |
| **Film-coated tablet weight (mg)** | **363.000** |

**Example F33:** inventive pharmaceutical film-coated tablet composition comprising lactose monohydrate, microcrystalline cellulose, sodium starch glycolate, croscarmellose sodium and magnesium stearate in extragranular phase and 96.25 mg eltrombopag olamine (equivalent to 75 mg eltrombopag), microcrystalline cellulose, and polyvinyl pyrrolidone (povidone) in intragranular phase

| **Ingredients** | **F33** |
|---|---|
| **Stage-A: (Dry Mix)** | **(mg/unit)** |
| Eltrombopag Olamine, *In-house* | 96.251 |
| Cellulose, Microcrystalline *Ph.Eur.* (Comprecel M 102 D+) | 18.269 |
| Sodium starch glycolate Type-A *Ph.Eur.* (Explotab) | 7.000 |
| **Stage-B: (Binder Solution / Granulation)** | |
| Polyvinyl pyrrolidone, Povidone *Ph.Eur.* (Plasdone K29/32) | 10.000 |
| Water, purified *Ph.Eur.* | q.s. |
| **Stage-C (Blending)** | |
| Cellulose, Microcrystalline *Ph.Eur.* (Comprecel M 102 D+) | 54.000 |
| Lactose monohydrate *Ph.Eur.* (Flowlac 90) | 120.980 |
| Sodium starch glycolate Type-A *Ph.Eur.* (Explotab) | 28.000 |
| Croscarmellose sodium *Ph.Eur.* (Ac-Di-Sol) | 10.000 |
| **Stage-D (Blending & Lubrication)** | |
| Magnesium stearate *Ph.Eur.* | 3.500 |
| **Core Tablet weight (mg)** | **348.000** |
| **Stage-E (Film-coating)** | |
| Opadry^{®} Brown *In-house* | 15.000 |
| Water, Purified *Ph.Eur.* | q.s. |
| **Film-coated tablet weight (mg)** | **363.000** |

**Examples F34 to F37:** inventive pharmaceutical film-coated tablet composition comprising lactose monohydrate, microcrystalline cellulose, sodium starch glycolate and magnesium stearate in extragranular phase and 16 mg, 31.9 mg, 63.2 mg or 95.7 mg eltrombopag olamine (equivalent to 12.5 mg, 25 mg, 50 mg or 75 mg eltrombopag) in combination with microcrystalline cellulose, polyvinyl pyrrolidone and sodium starch glycolate in intragranular phase

| **Ingredients** | **F34** | **F35** | **F36** | **F37** |
|---|---|---|---|---|
| **Stage-A: (Dry Mix)** | **(mg/unit)** | **(mg/unit)** | **(mg/unit)** | **(mg/unit)** |
| Eltrombopag Olamine, *In-house* | 95.703 | 63.802 | 31.901 | 15.951 |
| Cellulose, Microcrystalline *Ph.Eur. (Comprecel M 101 D*+*)* | 18.000 | 12.000 | 6.000 | 3.000 |
| Sodium starch glycolate Type-A *Ph.Eur.* (Explotab) | 7.000 | 4.667 | 2.333 | 1.167 |
| **Stage-B (Binder solution)** | | | | |
| Povidone *Ph.Eur.* (Plasdone K29/32) | 10.000 | 6.667 | 3.333 | 1.667 |
| Water, Purified *Ph.Eur.* | q.s. | q.s. | q.s. | q.s. |
| **Stage-C (Blending)** | | | | |
| Cellulose, Microcrystalline *Ph.Eur. (*Avicel PH 105*)* | 54.000 | 54.000 | 54.000 | 27.000 |
| Lactose monohydrate *Ph.Eur.* (Flowlac 90) | 149.797 | 193.364 | 236.933 | 118.467 |
| Sodium starch glycolate Type-A *Ph.Eur.* (Explotab) | 10.000 | 10.000 | 10.000 | 5.000 |
| **Stage-D (Lubrication)** | | | | |
| Magnesium stearate *Ph.Eur.* | 3.500 | 3.500 | 3.500 | 1.750 |
| **Core Tablet weight (mg)** | **348.000** | **348.000** | **348.000** | **174.000** |
| **Stage-E (Film-coating)** | | | | |
| Opadry ^{®} II Brown 85F565229 *In-house (Polyvinyl alcohol-partially hydrolyzed, Macrogol 3350, Titanium dioxide, Talc, Iron oxide red, Iron oxide yellow, Iron oxide black)* | 15.000 | - | - | - |
| Opadry ^{®} II Brown 85F565230 *In-house (Polyvinyl alcohol-partially hydrolyzed, Macrogol 3350, Titanium dioxide, Talc, Iron oxide red, Iron oxide yellow, Iron oxide black)* | - | 15.000 | - | - |
| Opadry ^{®} White YS-1-7706-G *In-house (Hypromellose 2910, Macrogol 400, Titanium dioxide, Polysorbate 80)* | - | - | 15.000 | 7.500 |
| Water, Purified *Ph.Eur.* | q.s. | q.s. | q.s. | q.s. |
| **Film-coated tablet weight (mg)** | **363.000** | **363.000** | **363.000** | **181.500** |

### Part C: General Procedure for the wet granulation compression (lactose intragranular / eltrombopag olamine extragranular)

### Stage A: (Dry Mix / Wet Granulation)

1. Stage-A materials were sifted through #35 mesh.
2. Sifted materials of step-1 loaded into high shear mixer granulator and mixed for 5 minutes with impeller at slow speed.
3. Step-2 material was granulated with a suitable binder solution, e.g., water or an aqueous solution such as aqueous povidone solution, at impeller slow speed.
4. The granules of step-3 material were dried in a suitable equipment to get required loss on drying.
5. The dried granules of step-4 were milled using Quadro^{®} Comil equipped with suitable screen.

### Stage B: (Blending)

6. Extragranular Stage B materials were sifted through #35 mesh.
7. Dried milled granules of step 5 and sifted extragranular materials of step 6 were loaded into the blender and mixed for 10 min.

### Stage C: (Lubrication)

8. Lubrication material of Stage C, e.g., magnesium stearate, was sifted through #35 mesh and added to blend mixture of step 7 and lubricated for 5 minutes.
9. The blend mixture of step 8 was compressed into tablets on rotary tablet compression machine using suitable tooling and parameters.

### Stage D: Film-Coating

10. Core tablets of step 9 were coated in coating pan using corresponding Opadry^{®} suspension, e.g., aqueous Opadry^{®} brown suspension.

### Part D: Process of production of inventive pharmaceutical tablet compositions (lactose intragranular / eltrombopag olamine extragranular)

The following inventive pharmaceutical tablet composition **F38** is produced in accordance with the general procedure as set out in Part C above:
**Example F38:** inventive pharmaceutical film-coated tablet composition comprising 96.25 mg eltrombopag olamine (equivalent to 75 mg eltrombopag), microcrystalline cellulose, polyvinyl pyrrolidone (povidone) and magnesium stearate in extragranular phase and lactose monohydrate and polyvinyl pyrrolidone (povidone) in intragranular phase

| **Ingredients** | **F38** |
|---|---|
| **Stage-A: (Dry Mix / Wet Granulation)** | **(mg/unit)** |
| Lactose monohydrate *Ph.Eur.* (Flowlac 90) | 193.037 |
| Polyvinyl pyrrolidone, Povidone *Ph.Eur.* (Plasdone K29/32) | 4.800 |
| Water, purified *Ph.Eur.* | q.s. |
| **Stage-B (Blending)** | |
| Eltrombopag Olamine, *In-house* | 95.703 |
| Cellulose, Microcrystalline *Ph.Eur.* (Comprecel M 102 D+) | 18.160 |
| Sodium starch glycolate Type-A *Ph.Eur.* (Explotab) | 28.000 |
| Polyvinyl pyrrolidone, Povidone *Ph.Eur.* (Plasdone K29/32) | 4.800 |
| **Stage-C (Lubrication)** | |
| Magnesium stearate *Ph.Eur.* | 3.500 |
| **Core Tablet weight (mg)** | **348.000** |
| **Stage-D (Film-coating)** | |
| Opadry^{®} Brown *In-house* | 15.000 |
| Water, Purified *Ph.Eur.* | q.s. |
| **Film-coated tablet weight (mg)** | 363.000 |

### Part E: Stability and dissolution of inventive pharmaceutical tablet compositions

An Accelerated Stability Assessment Program (ASAP) for up to 2-3 weeks under open exposure, according to Waterman 2011 (Waterman KC, The application of the Accelerated Stability Assessment Program (ASAP) to quality by design (QbD) for drug product stability, AAPS PharmSciTech, Vol. 12, No. 3, September 2011*)* is usually applied for a quick estimation of chemical stability (cf. Table 1). A precise relative comparison of stability of two different compositions, or more precise shelf-life prediction compared with long-term and accelerated stability in commercial blister packaging can be conducted according to ICH guideline Q1A(R2) (cf. Table 2).

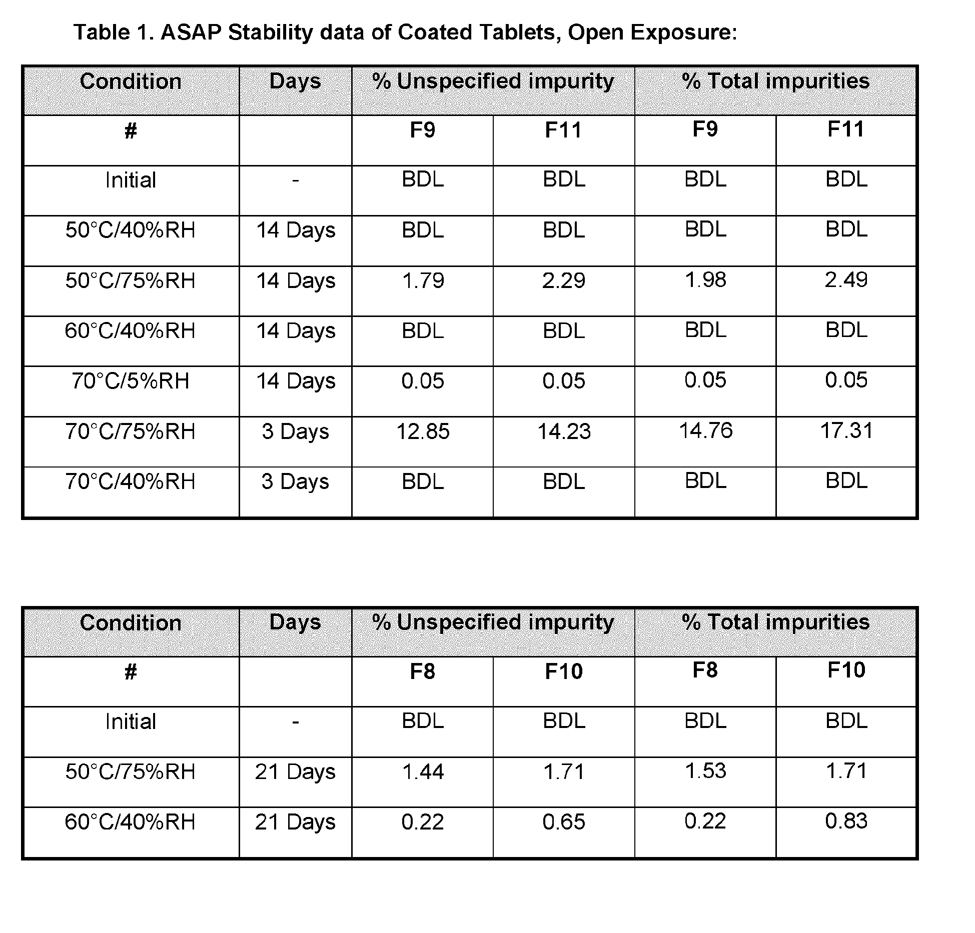

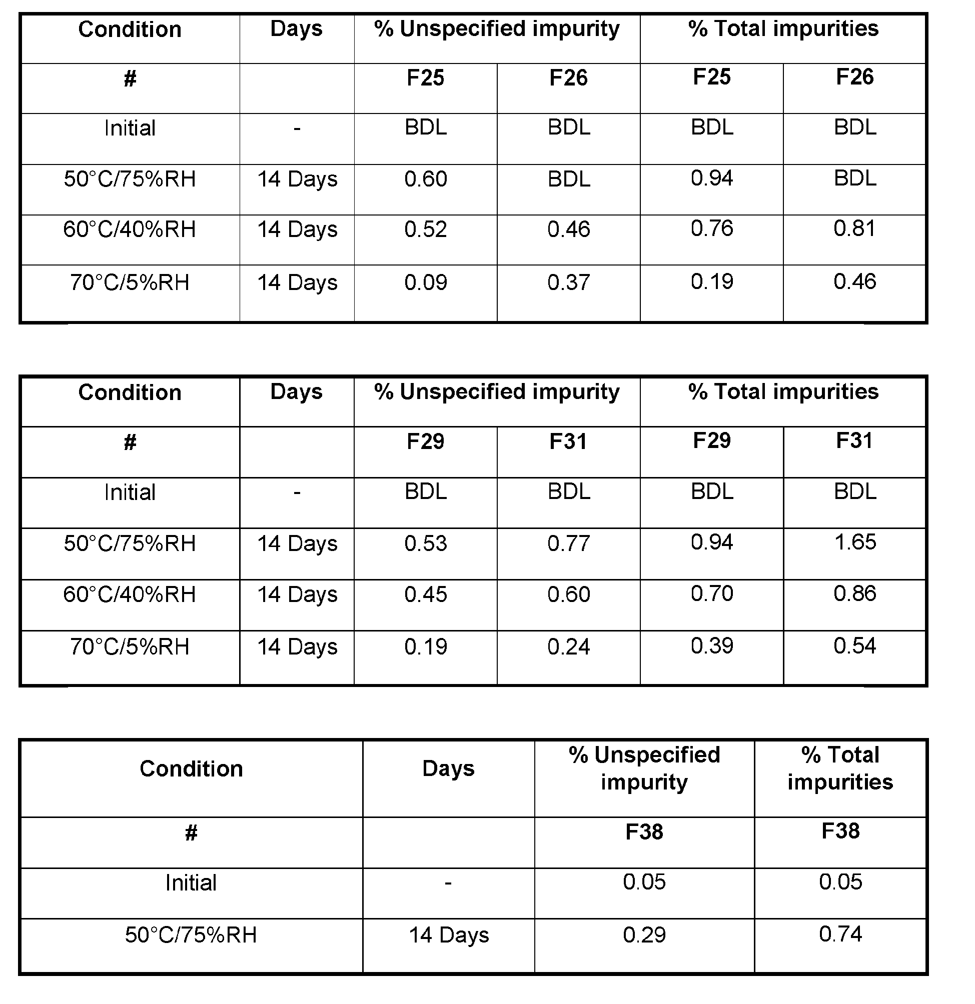

The amount of impurities measured for the inventive pharmaceutical tablet compositions initially after production are below detection limit (BDL). The impurities measured in the open exposure testing were found to be higher the higher the humidity was. At lower humidity impurities have not been detected even at higher temperatures.

The above data already suggests that inventive eltrombopag olamine tablets packaged preferably in Alu-Alu blister, will show less impurities at real time storage condition, as the water vapor transmission rate (WVTR) for Alu-Alu blister is reduced to the minimum. The finding is confirmed with the accelerated stability testings shown in table 2 below.

Accordingly, for tablets packaged in the commercially representative packaging material under accelerated storage conditions, which are also used in the Kapsi declaration, the degradation of eltrombopag olamine as active ingredient in the inventive pharmaceutical tablet compositions is decreased in comparison to the lactose containing tablet composition of the prior art (see Kapsi declaration), where the tablet composition already initially after production comprises 1.5 % total impurities.

**Table 2. Accelerated stability data of film-coated inventive tablets in Alu-Alu blister:**

| | | | | **40 °C / 75 % RH** | | | **50°C / 75 % RH** |
|---|---|---|---|---|---|---|---|
| **Composition** | **Specification** | **Impurities** | **Initial** | **1 month** | **2 month** | **6 month** | **15 days** |
| **F5** | Not more than 0.2 | **Unknown** | BDL | BDL | BDL | | |
| | Not more than 2.0 | **Total** | BDL | BDL | BDL | | |
| **F7** | Not more than 0.2 | **Unknown** | BDL | BDL | BDL | | |
| | Not more than 2.0 | **Total** | BDL | BDL | BDL | | |
| **F8** | Not more than 0.2 | **Unknown** | BDL | BDL | BDL | | |
| | Not more than 2.0 | **Total** | BDL | BDL | BDL | | |
| **F9** | Not more than 0.2 | **Unknown** | BDL | BDL | BDL | BDL | BDL |
| | Not more than 2.0 | **Total** | BDL | BDL | BDL | BDL | BDL |
| **F10** | Not more than 0.2 | **Unknown** | BDL | BDL | BDL | | |
| | Not more than 2.0 | **Total** | BDL | BDL | BDL | | |

| | | | | **40 °C / 75 % RH** | | | **50°C / 75 % RH** |
|---|---|---|---|---|---|---|---|
| **Composition** | **Specification** | **Impurities** | **Initial** | **1 month** | **2 month** | **6 month** | **15 days** |
| **F11** | Not more than 0.2 | **Unknown** | BDL | BDL | BDL | BDL | BDL |
| | Not more than 2.0 | **Total** | BDL | BDL | BDL | BDL | BDL |
| **F34** | Not more than 0.2 | **Unknown** | BDL | BDL | | | |
| | Not more than 2.0 | **Total** | BDL | BDL | | | |

The physical and chemical stability of the inventive pharmaceutical tablet composition may be tested in conventional manner, in particular at accelerated conditions according to ICH guideline Q1A(R2) (i.e. at 25 °C / 60 % relative humidity (RH) and/or at 40 °C / 75 % RH). The accelerated stability tests as set out in table 2 above have been performed according to applicable pharmaceutical regulatory standards as described e.g. in ICH or EMA guidelines and/or the European Pharmacopeia (EP), wherein the inventive pharmaceutical tablet composition have been packed in Alu-Alu blisters.

The amount of impurities measured for the inventive pharmaceutical tablet compositions initially after production are below detection limit. When using the regulatory accelerated stability assessment standards for Europe as storage conditions, the inventive pharmaceutical tablet compositions are sealed in Alu-Alu blisters and the amount of impurities is still below detection limit after one or two or six months storage. Accordingly, the inventive pharmaceutical tablet compositions show no degradation within six month accelerated storage.

The fact that no degradation products of eltrombopag olamine could be detected may be due to the separation of eltrombopag olamine in the intragranular phase and the reducing sugar, preferably lactose in the extragranular phase. When using higher amounts of reducing sugar, in particular lactose, the additional use of a polymeric binder agent, in particular povidone, in the intragranular phase and/or extragranular phase, in particular the extragranular phase and optionally the intragranular phase seems to be advantageous to further facilitate the decreased degradation of eltrombopag olamine in the inventive tablet composition.

**Table 3. Dissolution testing:**

| **Composition** | **Cumulative wt.% eltrombopag olamine after 45 minutes** |
|---|---|
| **F1** | **97** |
| **F3** | **96** |
| **F4** | **87** |
| **F9** | **94** |
| **F11** | **96** |

Dissolution testing according to tables 3-7 was performed using USP Apparatus II, 50 rpm, 900 ml of phosphate buffer pH 6.8 containing 0.5% Tween 80 as recommended for Eltrombopag Olamine in the FDA Dissolution Methods Database on the FDA webpage. Based on the Ph. Eur. (5.17.1) recommendation for immediate-release dosage forms, the specification is set as at least 75% of the active substance is dissolved within 45 minutes.

The experimental data of table 3 shows that 75 wt.% or more, preferably more than 80 wt.%, more preferably more than 90 wt.% of eltrombopag olamine of the inventive pharmaceutical tablet compositions is dissolved within 45 minutes in accordance with the regulatory standard test.

With respect to inventive eltrombopag tablet compositions F9 and F11, the test results could be confirmed after 1 month storage in Alu-Alu blister under accelerated condition at 40°C and 75 % relative humidity. With respect to inventive eltrombopag tablet composition F11, the test results could be confirmed also after 6 months storage in Alu-Alu blister under accelerated condition at 40°C and 75 % relative humidity. Accordingly, the inventive tablet compositions are not only stable over preferably 6 month, they also comply with the regulatory standards for dissolution testings, where at least 75 wt.% of eltrombopag olamine needs to be dissolved within 45 minutes.

In addition dissolution testings concerning, e.g. F17, F18, F20, F21, F23, and F25-F38, show that after 45 minutes at least 75 wt.%, preferably between 86 and 100 wt.% eltrombopag olamine based on the total weight of the eltrombopag olamine content in the tablet is dissolved.

With respect to inventive eltrombopag tablet composition F34, the test results could be confirmed after 1 month storage in Alu-Alu blister under accelerated condition at 40°C and 75 % relative humidity. With respect to inventive eltrombopag tablet composition F38, the test results could be confirmed after 14 days storage under open exposure stress condition at 50°C and 75 % relative humidity.

Furthermore, the dissolution of the inventive formulations of the present invention is comparable to the commercially available reference product Revolade^{®} film-coated tablets (FCTs), as shown below in tables 4, 5, 6, and 7 for the 75 mg, 50 mg, 25 mg, and 12.5 mg strength, correspondingly.

**Table 4. Comparative dissolution of Eltrombopag 75 mg FCTs with Revolade^{®} 75 mg:**

| **Composition** | **Cumulative wt.% eltrombopag olamine dissolved after 45 minutes** |
|---|---|
| **Revolade^{®} 75 mg (batch MX4H)** | **97** |
| **F25** | **93** |
| **F32** | **94** |
| **F33** | **95** |
| **F34** | **98** |
| **F38** | **100** |

**Table 5. Comparative dissolution of Eltrombopag 50 mg FCTs with Revolade^{®} 50 mg:**

| **Composition** | **Cumulative wt.% eltrombopag olamine dissolved after 45 minutes** |
|---|---|
| **Revolade^{®} 50 mg (batch 6Y6C)** | **94** |
| **F17** | **93** |
| **F26** | **99** |
| **F29** | **92** |
| **F35** | **97** |

**Table 6. Comparative dissolution of Eltrombopag 25 mg FCTs with Revolade^{®} 25 mg:**

| **Composition** | **Cumulative wt.% eltrombopag olamine dissolved after 45 minutes** |
|---|---|
| **Revolade^{®} 25 mg (batch 3B9C)** | **86** |
| **F18** | **91** |
| **F27** | **94** |
| **F30** | **94** |
| **F36** | **96** |

**Table 7. Comparative dissolution of Eltrombopag 12.5 mg FCTs with Revolade^{®} 12.5 mg:**

| **Composition** | **Cumulative wt.% eltrombopag olamine dissolved after 45 minutes** |
|---|---|
| **Revolade^{®} 12.5 mg (batch 9Y3C)** | **87** |
| **F20** | **88** |
| **F21** | **94** |
| **F23** | **92** |
| **F28** | **93** |
| **F31** | **91** |
| **F37** | **97** |

### EMBODIMENTS:

1. Pharmaceutical tablet composition comprising eltrombopag olamine as active ingredient and one or more pharmaceutically acceptable excipients including one or more reducing sugars, characterized in that the composition comprises or consists of
   either
   a) eltrombopag olamine in a therapeutically effective amount and one or more pharmaceutically acceptable excipients in an intragranular composition, and
   b) one or more reducing sugars, preferably selected from the group consisting of lactose, maltose, glucose, arabinose, and fructose and optionally further one or more pharmaceutically acceptable excipients in an extragranular composition,
      or
   c) one or more reducing sugars, preferably selected from the group consisting of lactose, maltose, glucose, arabinose, and fructose and optionally further one or more pharmaceutically acceptable excipients in an intragranular composition and
   d) eltrombopag olamine in a therapeutically effective amount and one or more pharmaceutically acceptable excipients in an extragranular composition.
2. Pharmaceutical tablet composition according to embodiment 1, wherein the reducing sugar in the extragranular composition comprises or consists of lactose selected from the group consisting of lactose monohydrate, anhydrous lactose, spray dried lactose and co-processed lactose.
3. Pharmaceutical tablet composition according to embodiment 1 or 2, wherein the reducing sugar content is greater than 5 wt.%, preferably at least 9 wt.%, more preferably in the range of 15 to 75 wt.% alternatively 40 to 70 wt.% respectively based on the total weight of the pharmaceutical tablet composition.
4. Pharmaceutical tablet composition according to any one of embodiments 1 to 3, wherein the further one or more pharmaceutically acceptable excipients in the intragranular and optionally extragranular composition are selected from suitable binder agents, preferably one or more binder agents are selected from the group consisting of povidone, copovidone, hydroxypropyl cellulose, low-substituted hydroxypropyl cellulose, hydroxypropyl methylcellulose, hydroxypropyl starch, polyethylene oxide, more preferably one or two binder agents are selected from povidone and copovidone or one or more binder agents are selected from the group consisting of povidone, copovidone, polyvinyl alcohol, polyethylene glycol, polyvinyl caprolactam-polyvinyl acetate-polyethylene glycol graft copolymer, polyethylene glycol-polyvinyl alcohol graft copolymer, hydroxypropyl cellulose, hydroxypropyl methyl cellulose, sodium carboxymethyl cellulose, and hydroxypropyl starch.
5. Pharmaceutical tablet composition according to any one of embodiments 1 to 4, wherein the further one or more pharmaceutically acceptable excipients in the intragranular and/or extragranular composition, preferably in the intragranular and extragranular composition are selected from suitable further diluent agents, preferably one or more further diluent agents are selected from the group consisting of erythritol, isomalt, maltitol, xylitol, microcrystalline cellulose, powdered cellulose, pregelatinized starch, starch, lactitol, mannitol, sorbitol, maltodextrin more preferably one, two or more further diluent agents are selected from erythritol, isomalt, maltitol, xylitol, and microcrystalline cellulose, even more preferably the further diluent agents are selected from microcrystalline cellulose and one, two or more selected from erythritol, isomalt, maltitol and xylitol, preferably xylitol.
6. Pharmaceutical tablet composition according to any one of embodiments 1 to 5 wherein the further one or more pharmaceutically acceptable excipients in the intragranular and/or extragranular composition, preferably in the extragranular composition are selected from suitable disintegrant agents, preferably one or more disintegrant agents are selected from the group consisting of sodium starch glycolate, crospovidone, and croscarmellose sodium, more preferably one or two selected from sodium starch glycolate and crospovidone or low-substituted hydroxypropyl cellulose.
7. Pharmaceutical tablet composition according to any one of embodiments 1 to 6, wherein the further one or more pharmaceutically acceptable excipients in the extragranular composition are selected from suitable lubricant agents, preferably selected from magnesium stearate, stearic acid, and sodium stearyl fumarate, more preferably magnesium stearate.
8. Pharmaceutical tablet composition according to any one of embodiments 1 to 7, wherein the pharmaceutical tablet composition is film coated with a suitable coating agent, preferably comprising one or more pharmaceutically acceptable polymers, optionally one or more pharmaceutically acceptable plasticizers, and optionally one or more pharmaceutically acceptable pigments.
9. Pharmaceutical tablet composition according to any one of embodiments 4 to 8, wherein
   i. the total content of binder agent(s) is at least 0.5 wt.% or more, more preferably is in the range of 0.5 to 7 wt.%, more preferably 1 to 3 wt.% and/or
   ii. the total content of diluent agent(s) including the reducing sugar amount is at least 5 wt.% or more, preferably is present in the range of 10 to 86 wt.%, alternatively in the range of 15 to 80 wt.%, alternatively in the range of 7 to 62 wt.%, alternatively in the range of 63 wt.% to 86 wt.% and/or
   iii. the total content of the disintegrant agent(s) is at least 3 wt.% or more, preferably is in the range of 4 to 15 wt.%, more preferably 5 to 14 wt.%, even more preferably 6 to 12 wt.% or alternatively is in the range of 3 to 5 wt.% and/or
   iv. the total content of the lubricant agent(s) is at least 0.5 wt.% or more, preferably is present in the range of 0.5 to 5 wt.%, preferably 0.8 to 3 wt.%, more preferably 0.9 to 1.5 wt.% and more preferably is 1 wt.% and/or
   v. the total content of the coating agent(s) is at least 2 wt.%, preferably in the range of 3 to 7 wt.%, more preferably 4 to 5 wt.%,
   respectively based on the total weight of the pharmaceutical tablet composition.
10. Pharmaceutical tablet composition according to any one of the preceding embodiments for use in the treatment or prophylaxis of immune (idiopathic) thrombocytopenic purpura (ITP), thrombocytopenia and/or acquired severe aplastic anaemia (SAA).
11. Process of production of a pharmaceutical tablet comprising eltrombopag olamine as active ingredient and one or more pharmaceutically acceptable excipients including one or more reducing sugars, characterized in that the process comprises the following steps:
   either
   a. mixing a therapeutically effective amount of eltrombopag olamine and one or more pharmaceutically acceptable excipients,
   b. wet granulation of components mixed in step a) with a suitable granulation fluid, preferably water or an aqueous solution of a binder, in a suitable amount to form an intragranular composition,
   c. mixing one or more reducing sugars, preferably selected from the group consisting of lactose, maltose, glucose, arabinose, and fructose and optionally further one or more pharmaceutically acceptable excipients to form an extragranular composition,
   d. mixing the extragranular composition of step c) with the intragranular composition of step b) to form a tablet composition, and
   e. compressing the tablet composition of step d) to form the pharmaceutical tablet composition comprising eltrombopag olamine as active ingredient in the intragranular phase and one or more reducing sugars in the extragranular phase,
      or
   f. mixing one or more reducing sugars, preferably selected from the group consisting of lactose, maltose, glucose, arabinose, and fructose, preferably lactose and optionally further one or more pharmaceutically acceptable excipients,
   g. wet granulation of components mixed in step f) with a suitable granulation fluid, preferably water or an aqueous solution of a binder, to form an intragranular composition,
   h. mixing a therapeutically effective amount of eltrombopag olamine and one or more pharmaceutically acceptable excipients to form an extragranular composition,
   i. mixing the extragranular composition of step h) with the intragranular composition of step g) to form a tablet composition, and
   j. compressing the tablet composition of step i) to form the pharmaceutical tablet composition comprising eltrombopag olamine as active ingredient in the extragranular phase and one or more reducing sugars in the intragranular phase.
12. Process according to embodiment 12, wherein the compressed pharmaceutical tablet composition of step d) is in a subsequent step e) film coated with one or more pharmaceutically acceptable excipients.
13. Pharmaceutical tablet composition comprising eltrombopag olamine as active ingredient and one or more pharmaceutically acceptable excipients including one or more reducing sugars, characterized in that the composition comprising or consisting of
   either
   a) eltrombopag olamine in a therapeutically effective amount and one or more pharmaceutically acceptable excipients in an intragranular composition, and
   b) one or more reducing sugars, preferably selected from the group consisting of lactose, maltose, glucose, arabinose, and fructose and optionally further one or more pharmaceutically acceptable excipients in an extragranular composition
      or
   c) one or more reducing sugars, preferably selected from the group consisting of lactose, maltose, glucose, arabinose, and fructose and optionally further one or more pharmaceutically acceptable excipients in an intragranular composition and
   d) eltrombopag olamine in a therapeutically effective amount and one or more pharmaceutically acceptable excipients in an extragranular composition
   is obtainable by the production process according to embodiment 11 or 12.
14. Use of a pharmaceutical tablet composition according to any one of embodiments 1 to 9 or 13, wherein the pharmaceutical tablet composition is used in the manufacture of a medicament for the treatment or prophylaxis of immune (idiopathic) thrombocytopenic purpura (ITP), thrombocytopenia and/or acquired severe aplastic anaemia (SAA).
15. A method of treatment or prophylaxis of immune (idiopathic) thrombocytopenic purpura (ITP), thrombocytopenia and/or acquired severe aplastic anaemia (SAA), comprising administering to a patient in need a pharmaceutical tablet composition in accordance with any one of embodiments 1 to 9 or 13.

## Claims

1. Pharmaceutical tablet composition comprising eltrombopag olamine as active ingredient and one or more pharmaceutically acceptable excipients including one or more reducing sugars, **characterized in that** the composition comprises or consists of
either
e) eltrombopag olamine in a therapeutically effective amount and one or more pharmaceutically acceptable excipients in an intragranular composition, and
f) one or more reducing sugars, preferably selected from the group consisting of lactose, maltose, glucose, arabinose, and fructose and optionally further one or more pharmaceutically acceptable excipients in an extragranular composition,
or
g) one or more reducing sugars, preferably selected from the group consisting of lactose, maltose, glucose, arabinose, and fructose and optionally further one or more pharmaceutically acceptable excipients in an intragranular composition and
h) eltrombopag olamine in a therapeutically effective amount and one or more pharmaceutically acceptable excipients in an extragranular composition.

2. Pharmaceutical tablet composition according to claim 1, wherein the reducing sugar in the extragranular composition comprises or consists of lactose selected from the group consisting of lactose monohydrate, anhydrous lactose, spray dried lactose and co-processed lactose.

3. Pharmaceutical tablet composition according to claim 1 or 2, wherein the reducing sugar content is greater than 5 wt.%, preferably at least 9 wt.%, more preferably in the range of 15 to 75 wt.% alternatively 40 to 70 wt.% respectively based on the total weight of the pharmaceutical tablet composition.

4. Pharmaceutical tablet composition according to any one of claims 1 to 3, wherein the further one or more pharmaceutically acceptable excipients in the intragranular and optionally extragranular composition are selected from suitable binder agents, preferably one or more binder agents are selected from the group consisting of povidone, copovidone, hydroxypropyl cellulose, low-substituted hydroxypropyl cellulose, hydroxypropyl methylcellulose, hydroxypropyl starch, polyethylene oxide, more preferably one or two binder agents are selected from povidone and copovidone or one or more binder agents are selected from the group consisting of povidone, copovidone, polyvinyl alcohol, polyethylene glycol, polyvinyl caprolactam-polyvinyl acetate-polyethylene glycol graft copolymer, polyethylene glycol-polyvinyl alcohol graft copolymer, hydroxypropyl cellulose, hydroxypropyl methyl cellulose, sodium carboxymethyl cellulose, and hydroxypropyl starch.

5. Pharmaceutical tablet composition according to any one of claims 1 to 4, wherein the further one or more pharmaceutically acceptable excipients in the intragranular and/or extragranular composition, preferably in the intragranular and extragranular composition are selected from suitable further diluent agents, preferably one or more further diluent agents are selected from the group consisting of erythritol, isomalt, maltitol, xylitol, microcrystalline cellulose, powdered cellulose, pregelatinized starch, starch, lactitol, mannitol, sorbitol, maltodextrin more preferably one, two or more further diluent agents are selected from erythritol, isomalt, maltitol, xylitol, and microcrystalline cellulose, even more preferably the further diluent agents are selected from microcrystalline cellulose and one, two or more selected from erythritol, isomalt, maltitol and xylitol, preferably xylitol.

6. Pharmaceutical tablet composition according to any one of claims 1 to 5 wherein the further one or more pharmaceutically acceptable excipients in the intragranular and/or extragranular composition, preferably in the extragranular composition are selected from suitable disintegrant agents, preferably one or more disintegrant agents are selected from the group consisting of sodium starch glycolate, crospovidone, and croscarmellose sodium, more preferably one or two selected from sodium starch glycolate and crospovidone or low-substituted hydroxypropyl cellulose.

7. Pharmaceutical tablet composition according to any one of claims 1 to 6, wherein the further one or more pharmaceutically acceptable excipients in the extragranular composition are selected from suitable lubricant agents, preferably selected from magnesium stearate, stearic acid, and sodium stearyl fumarate, more preferably magnesium stearate.

8. Pharmaceutical tablet composition according to any one of claims 1 to 7, wherein the pharmaceutical tablet composition is film coated with a suitable coating agent, preferably comprising one or more pharmaceutically acceptable polymers, optionally one or more pharmaceutically acceptable plasticizers, and optionally one or more pharmaceutically acceptable pigments.

9. Pharmaceutical tablet composition according to any one of claims 4 to 8, wherein
i. the total content of binder agent(s) is at least 0.5 wt.% or more, more preferably is in the range of 0.5 to 7 wt.%, more preferably 1 to 3 wt.% and/or
ii. the total content of diluent agent(s) including the reducing sugar amount is at least 5 wt.% or more, preferably is present in the range of 10 to 86 wt.%, alternatively in the range of 15 to 80 wt.%, alternatively in the range of 7 to 62 wt.%, alternatively in the range of 63 wt.% to 86 wt.% and/or
iii. the total content of the disintegrant agent(s) is at least 3 wt.% or more, preferably is in the range of 4 to 15 wt.%, more preferably 5 to 14 wt.%, even more preferably 6 to 12 wt.% or alternatively is in the range of 3 to 5 wt.% and/or
iv. the total content of the lubricant agent(s) is at least 0.5 wt.% or more, preferably is present in the range of 0.5 to 5 wt.%, preferably 0.8 to 3 wt.%, more preferably 0.9 to 1.5 wt.% and more preferably is 1 wt.% and/or
v. the total content of the coating agent(s) is at least 2 wt.%, preferably in the range of 3 to 7 wt.%, more preferably 4 to 5 wt.%,
respectively based on the total weight of the pharmaceutical tablet composition.

10. Pharmaceutical tablet composition according to any one of the preceding claims for use in the treatment or prophylaxis of immune (idiopathic) thrombocytopenic purpura (ITP), thrombocytopenia and/or acquired severe aplastic anaemia (SAA).

11. Process of production of a pharmaceutical tablet comprising eltrombopag olamine as active ingredient and one or more pharmaceutically acceptable excipients including one or more reducing sugars, **characterized in that** the process comprises the following steps:
either
k. mixing a therapeutically effective amount of eltrombopag olamine and one or more pharmaceutically acceptable excipients,
I. wet granulation of components mixed in step a) with a suitable granulation fluid, preferably water or an aqueous solution of a binder, in a suitable amount to form an intragranular composition,
m. mixing one or more reducing sugars, preferably selected from the group consisting of lactose, maltose, glucose, arabinose, and fructose and optionally further one or more pharmaceutically acceptable excipients to form an extragranular composition,
n. mixing the extragranular composition of step c) with the intragranular composition of step b) to form a tablet composition, and
o. compressing the tablet composition of step d) to form the pharmaceutical tablet composition comprising eltrombopag olamine as active ingredient in the intragranular phase and one or more reducing sugars in the extragranular phase,
or
p. mixing one or more reducing sugars, preferably selected from the group consisting of lactose, maltose, glucose, arabinose, and fructose, preferably lactose and optionally further one or more pharmaceutically acceptable excipients,
q. wet granulation of components mixed in step f) with a suitable granulation fluid, preferably water or an aqueous solution of a binder, to form an intragranular composition,
r. mixing a therapeutically effective amount of eltrombopag olamine and one or more pharmaceutically acceptable excipients to form an extragranular composition,
s. mixing the extragranular composition of step h) with the intragranular composition of step g) to form a tablet composition, and
t. compressing the tablet composition of step i) to form the pharmaceutical tablet composition comprising eltrombopag olamine as active ingredient in the extragranular phase and one or more reducing sugars in the intragranular phase.

12. Process according to claim 12, wherein the compressed pharmaceutical tablet composition of step d) is in a subsequent step e) film coated with one or more pharmaceutically acceptable excipients.

13. Pharmaceutical tablet composition comprising eltrombopag olamine as active ingredient and one or more pharmaceutically acceptable excipients including one or more reducing sugars, **characterized in that** the composition comprising or consisting of
either
e) eltrombopag olamine in a therapeutically effective amount and one or more pharmaceutically acceptable excipients in an intragranular composition, and
f) one or more reducing sugars, preferably selected from the group consisting of lactose, maltose, glucose, arabinose, and fructose and optionally further one or more pharmaceutically acceptable excipients in an extragranular composition
or
g) one or more reducing sugars, preferably selected from the group consisting of lactose, maltose, glucose, arabinose, and fructose and optionally further one or more pharmaceutically acceptable excipients in an intragranular composition and
h) eltrombopag olamine in a therapeutically effective amount and one or more pharmaceutically acceptable excipients in an extragranular composition
is obtainable by the production process according to claim 11 or 12.
